(19) Europäisches Patentamt
European Patent Office
Office européen des brevets

(11) Publication number: **0 092 037 A1**

(12) # EUROPEAN PATENT APPLICATION

(21) Application number: **83101634.0**

(22) Date of filing: **19.02.83**

(51) Int. Cl.³: **C 07 H 15/20**, C 07 H 3/04, A 61 K 31/70, A 61 K 7/16

(30) Priority: **16.04.82 US 369047**
**16.04.82 US 369048**
**16.04.82 US 369049**
**24.05.82 US 380986**

(43) Date of publication of application: **26.10.83**
**Bulletin 83/43**

(84) Designated Contracting States: **BE CH DE FR GB IT LI NL SE**

(71) Applicant: **AMERICAN CYANAMID COMPANY, 1937 West Main Street P.O. Box 60, Stamford Connecticut 06904 (US)**

(72) Inventor: **Schaub, Robert Eugene, 80 Hillcrest Drive, Upper Saddle River New Jersey 07458 (US)**
Inventor: **Upeslacis, Janis, 3 Keim Drive, Pomona New York 10970 (US)**
Inventor: **Bernstein, Seymour, 26 Scott Drive, New City New York 10956 (US)**

(74) Representative: **Wächtershäuser, Günter, Dr., Tal 29, D-8000 München 2 (DE)**

(54) **Glucopyranosyl sulfates as modulators of the complement system.**

(57) Novel compounds of the formula:

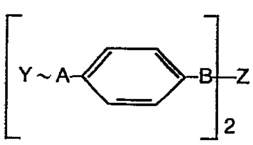
(∼ = α or β)

wherein Y is

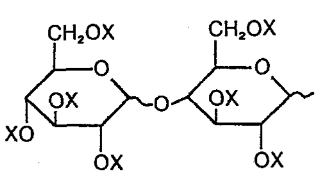
or

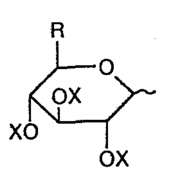
.

EP 0 092 037 A1

TITLE MODIFIED
see front page

TITLE: MODULATORS OF THE COMPLEMENT SYSTEM

This invention relates to novel compounds of the following formula:

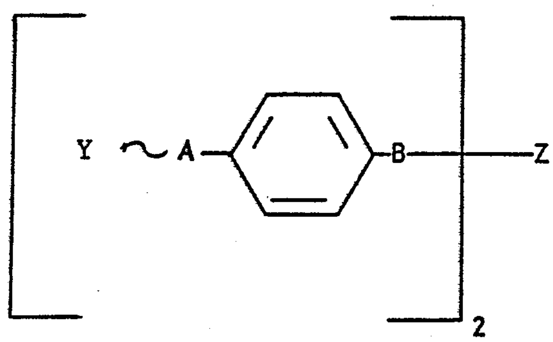 (~ = α or β)

I

wherein Y is 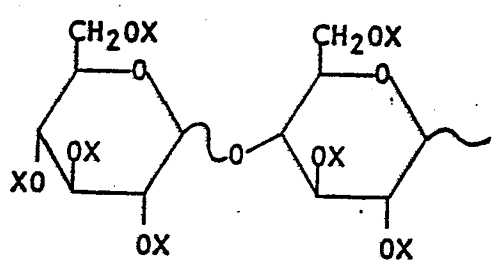

or 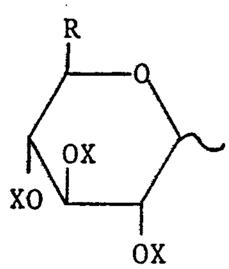 ;

X is $-SO_3M$ and M is a nontoxic pharmaceutically acceptable cation salt, wherein the salt forming moiety is alkali metal, alkaline earth metal, aluminum, ammonia or substituted ammonia consisting of trialkylamine ($C_1$-$C_6$), piperidine, pyrazine, alkanolamine ($C_2$-$C_6$) or cycloalkylamine ($C_3$-$C_6$); A is -S- or -O-; B is -NHCO-, $-NHSO_2-$, $-NHCH_2-$ or -NH-; R is $CH_2OSO_3M$, $COOCH_3$ or COOM; and Z is -CO- or a straight or branched chain alkylidene group $-(CH_2)_m-$, where m is an integer 0-12, inclusive, with the proviso that when m is zero or one, B cannot be $-NHSO_2-$, which compounds are highly active as complement modulators.

Although the compounds of Formula I are shown as being fully sulfated, this invention contemplates partially sulfated products.

The invention further relates to novel compounds of the following formula:

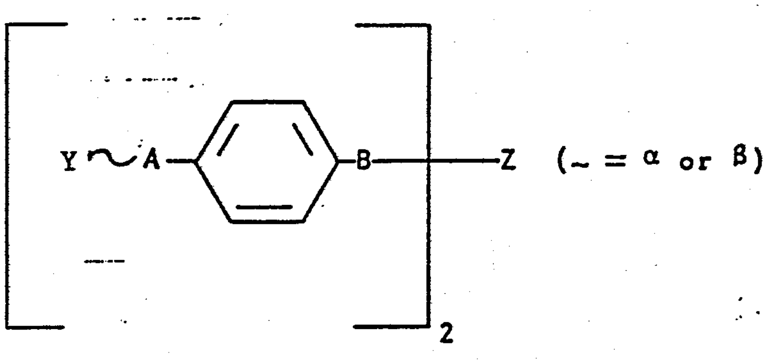

II

wherein Y is

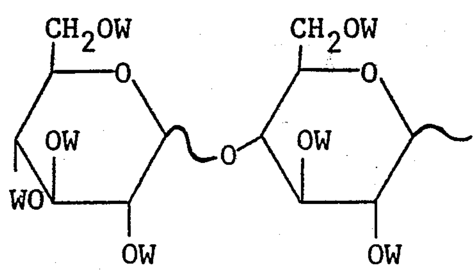

or 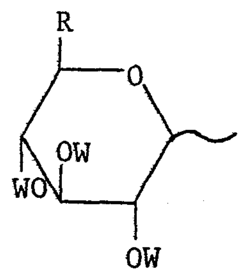 ;

A, B and Z are as described in Formula I; W is -H or $-COCH_3$; and R is $-CH_2OW$; -COOH or $-COOCH_3$.

In the above Formulas I and II the invention is intended to include disaccharides consisting of aldohexoses, ketohexoses, aldopentoses and the like as well as oligosaccharides wherein the sugar units are 2-8 consisting of maltotrioses, maltotetraoses, maltopentaoses and the like.

The term "complement" refers to a complex group of proteins in body fluids that, working together with antibodies or other factors, play an important role as mediators of immune, allergic, immunochemical and/or immunopathological reactions. The reactions in which complement participates take place in blood serum or in other body fluids, and hence are considered to be humoral reactions.

With regard to human blood, there are at present more than 20 proteins in the complement system consisting of the so-called classical and alternative pathways. These complement proteins are generally designated by the letter C and by number: C1, C2, C3 and so on up to C9. The complement protein C1 is actually an assembly of subunits designated C1*q*, C1*r* and C1*s*. The numbers assigned to the complement proteins reflect the sequence in which they become active, with the exception of complement protein C4, which reacts after C1 and before C2. The numerical assignments for the proteins in the complement system were made before the reaction sequence was fully understood. A more detailed discussion of the complement system and its biochemical, biological and pathological role in the body processes can be found in, for example, Bull. W. H. O. 39: 935 (1968); Annu. Rev. Med. 19: 1 (1968); Johns Hopkins

Med. J. 128: 57 (1971); Harvey Lect. 66: 75 (1972); N. Engl. J. Med. 287: 452, 489, 545, 592, 642 (1972); Sci. Am. 229 (5): 54 (1973); Fed. Pro. 32: 134 (1973); Med. World, October 11, 1974, p. 53; J. Allergy Clin. Immunol. 53: 298 (1974); Cold Spring Harbor Conf. Cell Proliferation 2/Proteases Biol. Control: 229 (1975); Annu. Rev. Biochem. 44: 697 (1975); Complement in Clinical Medicine, Dis. Mon. (1975); Complement, Scope, December 1975; Ann. Intern. Med. 84: 580 (1976); Transplant Rev.: 32 (1976); "Complement: Mechanisms and Functions," Prentice-Hall, Englewood Cliffs, N. J. (1976); Essays Med. Biochem. 2: 1 (1976); Hosp. Pract. 12: 33 (1977); Perturbation of Complement in Disease, Chap. 15 in Biol. Amplification Systems in Immunol. (Ed. Day and Good), Plenum, New York and London (1977); Am. J. Clin. Pathol. 68: 647 (1977); Biochem. Soc. Trans. 5: 1659 (1977); Harvey Lect. 72: 139 (1976-1977); J. Periodontol. 48: 505 (1977); Biochem. Soc. Trans. 6: 798 (1978); Clin. and Exp. Dermatol. 4: 271 (1979); Infect. Dis. Rev. 1: 483 (1979).

The complement system (e.g., classical pathway) can be considered to consist of three subsystems: (1) a recognition unit (Clq) which enables it to combine with antibody molecules that have detected a foreign invader; (2) an activation unit (Clr, Cls, C2, C4, C3) which prepares a site on the neighboring membrane; and (3) an attack unit (C5, C6, C7, C8 and C9) which creates a "hole" in the membrane. The membrane attack unit is nonspecific; it destroys invaders only because it is generated in their neighborhood. In order to minimize damage to the host's own cells, its activity must be limited in time. This limitation is accomplished partly by the spontaneous decay of activated complement and partly by interference by inhibitors and destructive enzymes. The control of complement, however, is not perfect, and there are times when damage is done to host's cells. Immunity is, therefore, a double-edged sword.

Activation of the complement system also accelerates blood clotting. This action comes about by way of the complement-mediated release of a clotting factor from platelets. The biologically active complement fragments and complexes can become involved in reactions that damage the host's cells. These pathogenic reactions can result in the development of immune-complex diseases. For example, in some forms of nephritis, complement damages the basal membrane of the kidney, resulting in the escape of protein from the blood into the urine. The disease disseminated lupus erythematosus belongs in this category; its symptoms include nephritis, visceral lesions and skin eruptions. The treatment of diphtheria or tetanus with the injection of large amounts of antitoxin sometimes results in serum sickness, an immune-complex disease. Rheumatoid arthritis also involves immune complexes. Like disseminated lupus erythematosus, it is an autoimmune disease in which the disease symptoms are caused by pathological effects of the immune system in the host's tissues. In summary, the complement system has been shown to be involved with inflammation, coagulation, fibrinolysis, antibody-antigen reactions and other metabolic processes.

In the presence of antibody-antigen complexes the complement proteins are involved in a series of reactions which may lead to irreversible membrane damage if they occur in the vicinity of biological membranes. Thus, while complement constitutes a part of the body's defense mechanism against infection it also results in inflammation and tissue damage in the immunopathological process. The nature of certain complement proteins, suggestions regarding the mode of complement binding to biological membranes and the manner in which complement effects membrane damage are discussed in Annu. Rev. Biochem. 38: 389 (1969); J. Exp. Med. 141: 724 (1975); J. Immunol. 116: 1431 (1976); 119: 1, 1195, 1358, 1482 (1977); 120: 1841 (1978); Immunochemistry 115: 813

(1978); J. Biol. Chem. 254: 9908 (1979).

A variety of substances have been disclosed as inhibiting the complement system, i.e., as complement inhibitors. For example, the compounds, 3,3'-ureylenebis-[6-(2-amino-8-hydroxy-6-sulfo-1-naphthylazo)benzenesulfonic acid], tetrasodium salt (chlorazol fast pink), heparin and a sulphated dextran have been reported to have an anticomplementary effect, Br. J. Exp. Pathol. 33: 327 (1952). German Patent No. 2,254,893 or South African Patent No. 727,923 discloses certain 1-(diphenylmethyl)-4-(3-phenylallyl)piperazines useful as complement inhibitors. Other chemical compounds having complement inhibiting activity are disclosed in, for example, J. Med. Chem. 12: 415, 902, 1049, 1053 (1969); Can. J. Biochem. 47: 547 (1969); J. Immunol. 104: 279 (1970); J. Immunol. 106: 241 (1971); J. Immunol. 111: 1061 (1973); Biochim. Biophys. Acta 317: 539 (1973); Life Sci. 13: 351 (1973); J. Immunol. 113: 584 (1974); Immunology 26: 819 (1974); J. Med. Chem. 17: 1160 (1974); Biochim. Biophys. Res. Comm. 67: 225 (1975); Ann. N. Y. Acad. Sci. 256: 441 (1975); J. Med. Chem. 19: 634, 1079 (1976); J. Immunol. 118: 466 (1977); Arch. Int. Pharmacodyn. 226: 281 (1977); Biochem. Pharmacol. 26: 325 (1977); J. Pharm. Sci. 66: 1367 (1977); Chem. Pharm. Bull. 25: 1202 (1977); Biochim. Biophys. Acta 484: 417 (1977); J. Clin. Microbiol. 5: 278 (1977); Immunochemistry 15: 231 (1978); Immunology 34: 509 (1978); J. Exp. Med. 147: 409 (1978); Thromb. Res. 14: 179 (1979); J. Immunol. 122: 2418 (1979); J. Chem. Soc. Chem. Comm. 726 (1979); Immunology 36: 131 (1979); Biochim. Biophys. Acta 611: 196 (1980); and J. Med. Chem. 23: 240 (1980).

It has been reported that the known complement inhibitors, epsilon-aminocaproic acid and tranexamic acid, have been used with success in the treatment of hereditary angioneurotic edema, a disease state resulting from an inherited deficiency or lack of function of the serum

inhibitor of the activated first component of complement (Cl inhibitor), N. Engl. J. Med. 286: 808 (1972); 287: 452 (1972); Ann. Intern. Med. 84: 580 (1976); J. Allergy Clin. Immunol. 60: 38 (1977). Also androgenic steroids have been used successfully in the treatment of this physiological disorder; see Medicine 58: 321 (1979); Arthritis Rheum. 22: 1295 (1979); Am. J. Med. 66: 681 (1979); and J. Allergy Clin. Immunol. 65: 75 (1980).

It has also been reported that the drug pentosan-polysulfoester has an anticomplementary activity on human serum, both in vitro and in vivo, as judged by the reduction in total hemolytic complement activity, Pathol. Biol. 25: 33; 25 (2): 105; 25 (3): 179 (1977).

The compounds of Formula I find utility as complement modulators in body fluids and as such may be used to ameliorate or prevent those pathological reactions requiring the function of complement and in the therapeutic treatment of warm-blooded animals having immunologic diseases such as rheumatoid arthritis, systemic lupus erythematosus, certain kinds of glomerulonephritis, certain kinds of autoallergic hemolytic anemia, certain kinds of platelet disorders and certain kinds of vasculitis. These compounds may also be used in the therapeutic treatment of warm-blooded animals having nonimmunologic diseases such as paroxysmal nocturnal hemoglobinurea, hereditary angioneurotic edema and inflammatory states induced by the action of bacterial or lysosomal enzymes on the appropriate complement components as, for example, inflammation following coronary occlusion. They also may be useful in the treatment of transplant rejection and ulcers and as blood culture and transport mediums. The sulfated compounds of this invention such as the sodium and aluminum salts, may be particularly useful in the treatment of ulcers and the like on oral therapy. Also, the non-sulfated intermediate compounds of Formula II may be useful as immuno-enhancing agents or potentiators.

The novel compounds of this invention may be prepared according to the following flowcharts.

FLOWCHART A

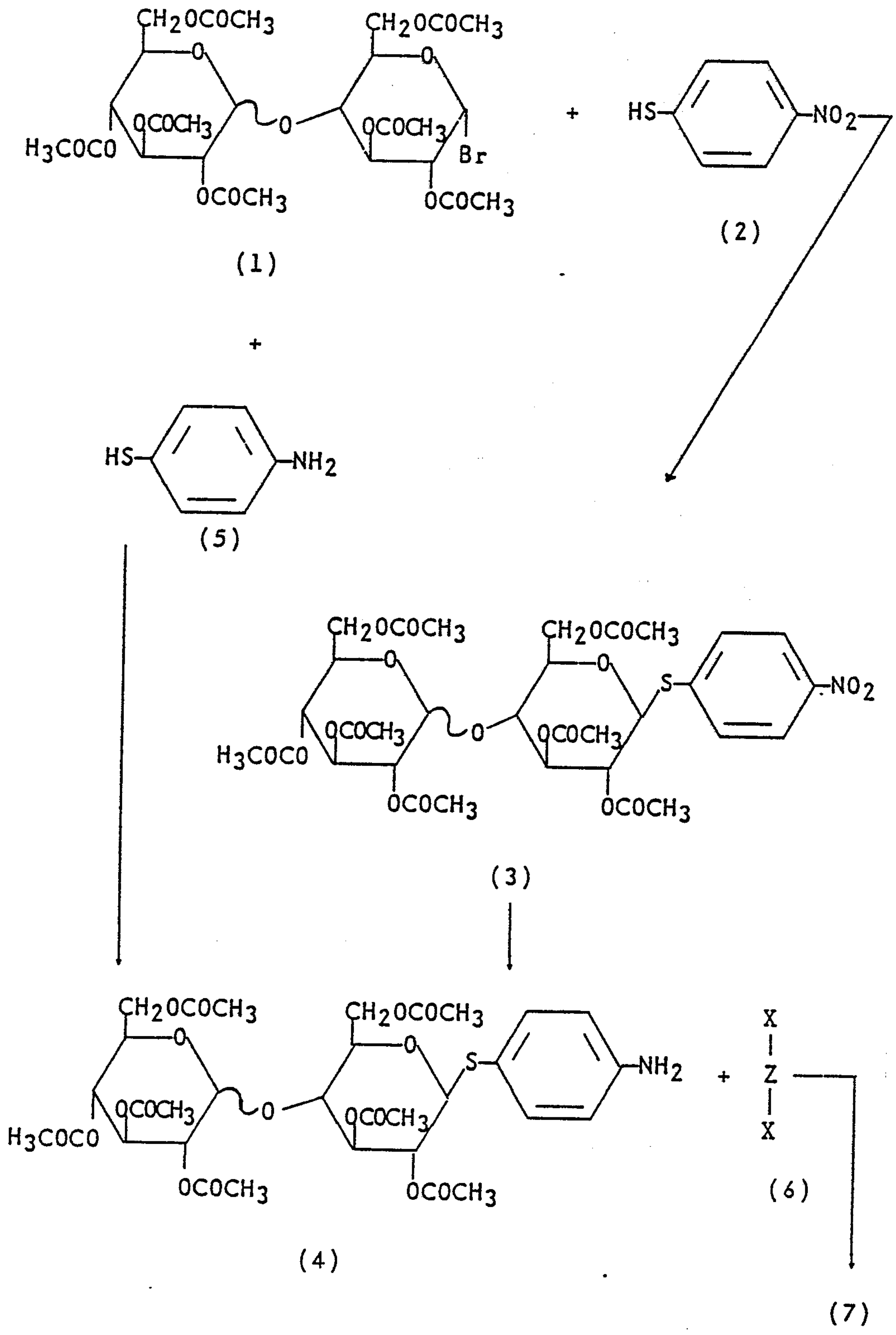

(1) + (2)

(5)

(3)

(4) + (6)

(7)

FLOWCHART A (continued)

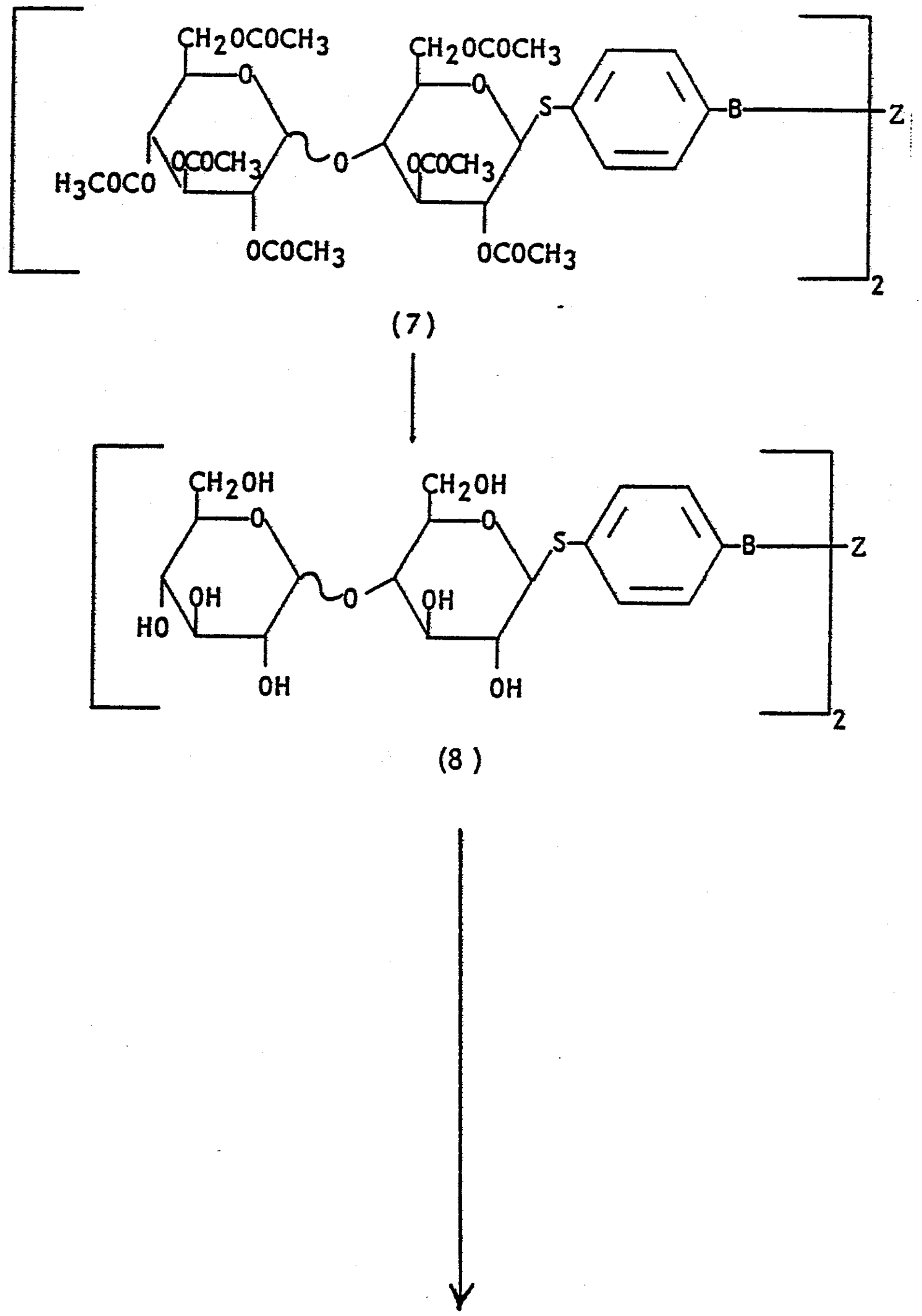

(7)

(8)

(9)

FLOWCHART A (continued)

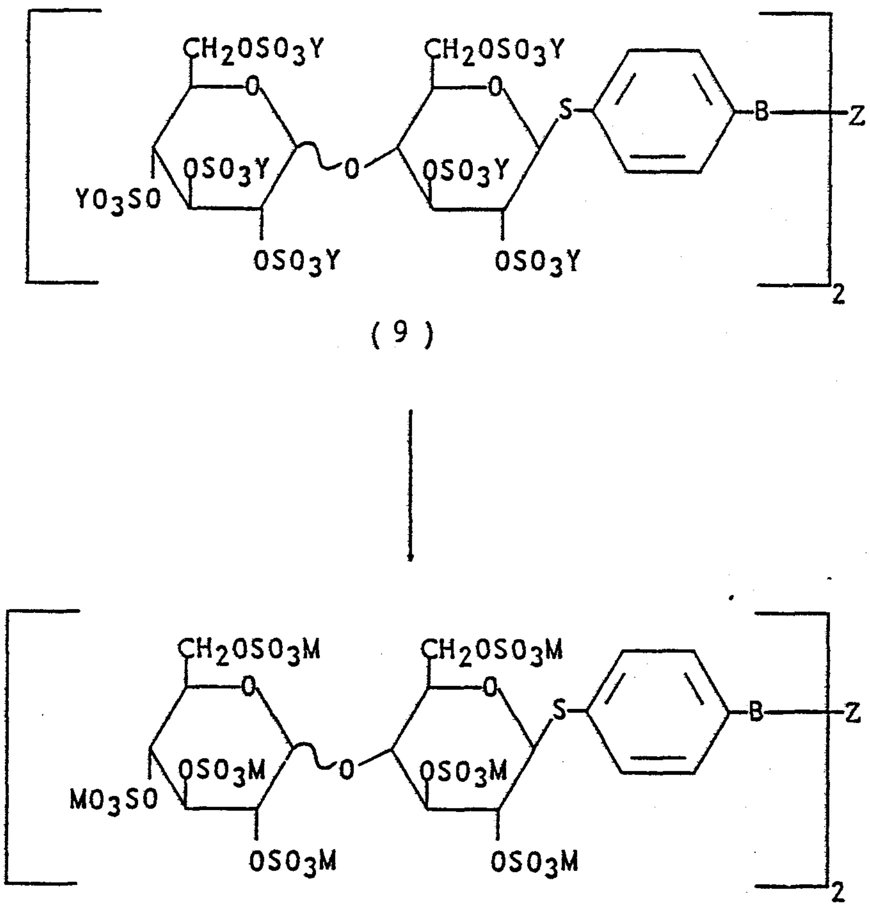

(9)

(10)

In accordance with the above Flowchart A, a brominated peracetyl sugar (1) is reacted with a p-nitrothio phenol (2), and sodium hydride in a solvent such as dimethoxyethane under an inert atmosphere for several hours, giving a 4-nitrophenyl 2,3,6-tri-O-acetyl-4-O-(2,3,4,6-tetra-O-acetyl-α(or β)-D-glucopyranosyl)-1-thio-α(or β)-D-glucopyranoside (3) which is then catalytically reduced to the corresponding 4-aminophenyl derivative (4). Alternatively, similar reaction of (1) with p-aminothiophenol (5) produces (4) directly without requiring reduction. The derivative (4) is then treated with an acid chloride (6) where X is, for example, COCl or $SO_2Cl$; Z is $-(CH_2)_m-$ and m is an integer 0-12 in a solvent such as acetonitrile under an inert atmosphere for several hours giving a N,N'-bis[4-[2,3,6-tri-O-acetyl-4-O-(2,3,4,6-tetra-O-acetyl-α(or β)-D-glucopyranosyl)-α(or β)-D-glucopyranosylthio]phenyl]alkyldiamide (7), where Z is as described above and B is, for example, -NHCO-. The derivative (7) is then reacted with ammonia-saturated methanol at -5 to +5°C under an inert atmosphere or with sodium in methanol for several hours giving a N,N'-bis[4-[4-O-(α(or β)-D-glucopyranosyl)-α(or β)-D-glucopyranosylthio]phenyl]alkyldiamide (8) where B and Z are as described above. The derivative (7) may also be reduced with diborane in dry tetrahydrofuran under an inert atmosphere at -5 to +5°C, giving the derivative (8) where Z is as described above and B is $-NHCH_2-$. Derivative (8) is then reacted with triethylamine-sulfur trioxide complex in N,N-dimethylacetamide, under an inert atmosphere at 60-65°C for several hours, giving the tetradecatriethylammonium derivative (9), where Y is $\overset{+}{N}H(C_2H_5)_3$ which is then, if desired, reacted with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitated in ethanol, giving the end product

(10) of this invention.

FLOWCHART B

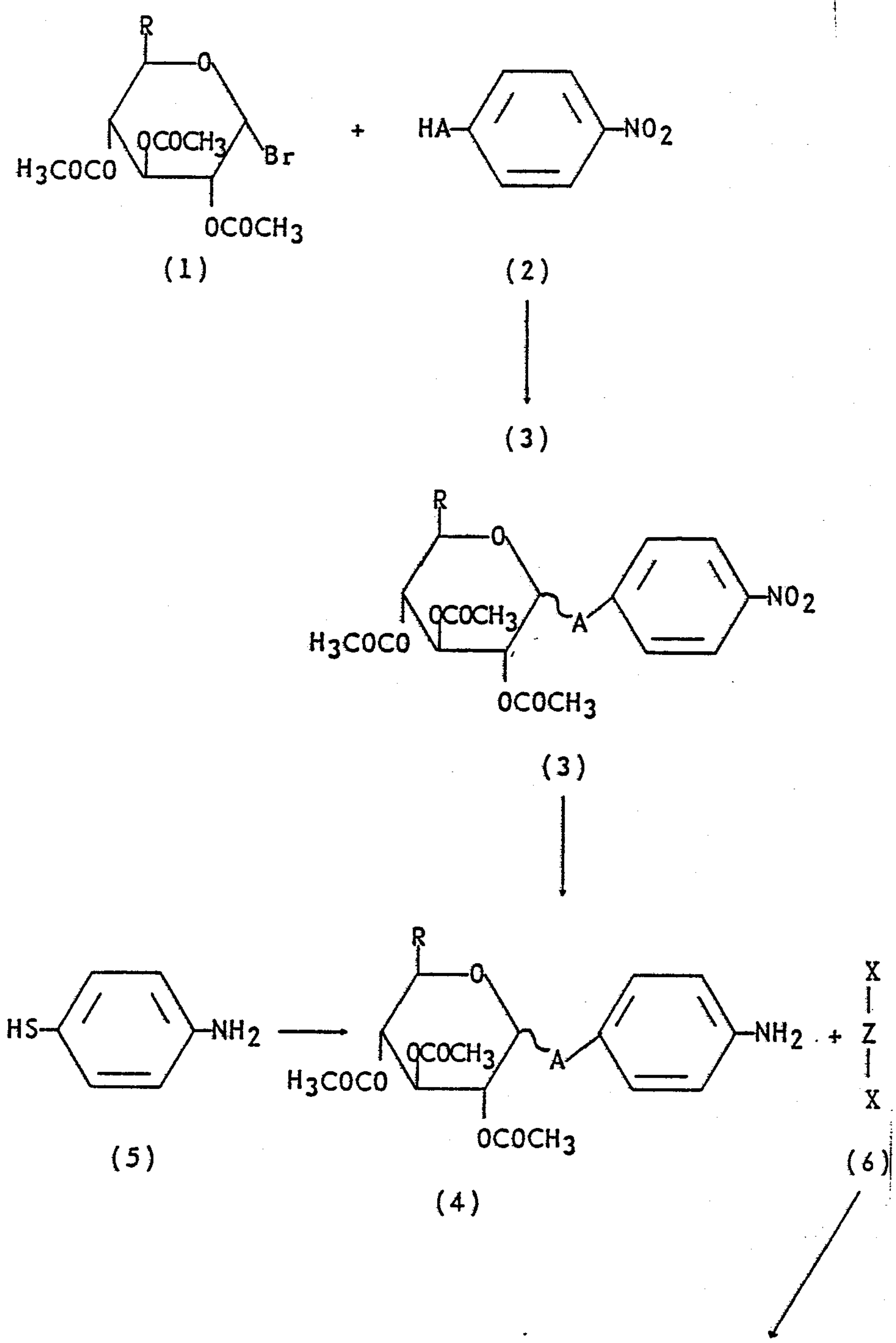

(1) (2) (3) (3) (5) (4) (6)

FLOWCHART B (continued)

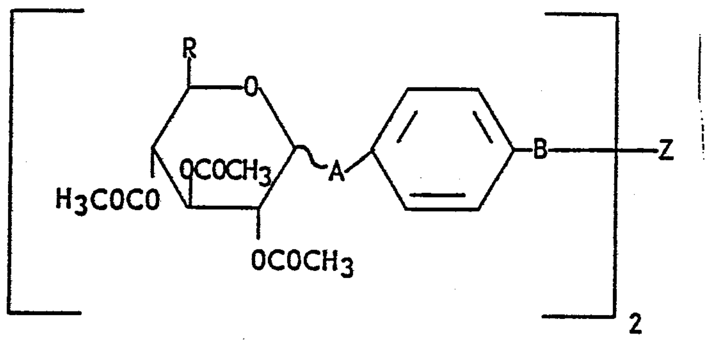

(7)

(8)

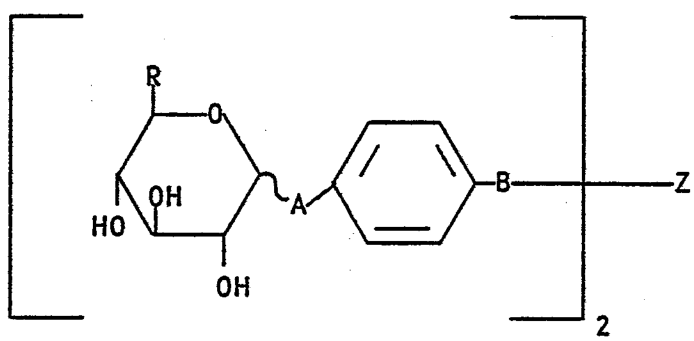

(8)

FLOWCHART B (continued)

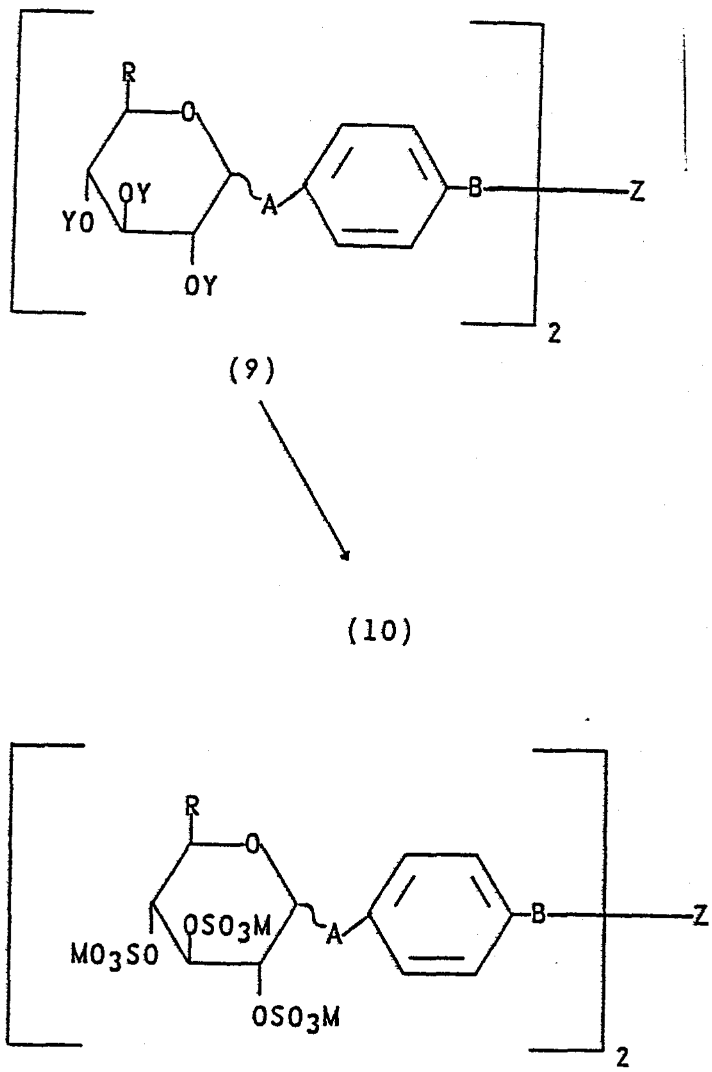

(9)

(10)

(10)

In accordance with the above Flowchart B, a bromoacetyl pyranoside (1) [where R is $CH_2OCOCH_3$ or $COOCH_3$] is reacted with a p-nitrothio(or oxy)phenyl (2) [where A is -S- or -O-] and sodium hydride in a solvent such as dimethoxyethane, under an inert atmosphere for several hours, giving a 4-nitrophenyl 2,3,4-tri-O-acetyl-α(or β)-D-glucopyranosyl)-1-thio(or oxy) derivative (3) which is then catalytically reduced to the corresponding 4-aminophenyl derivative (4). Alternatively, similar reaction of (1) with p-aminothiophenol (5) produces (4) directly without requiring reduction. The derivative (4) is then treated with an acid chloride (6) [where X is -COCl or $-SO_2Cl$; Z is $(CH_2)_m$; and m is an integer 0-12] in a solvent such as acetonitrile under an inert atmosphere for several hours, giving a N,N'-bis[4-[(2,3,4-tri-O-acetyl-α(or β)-D-glucopyranosyl)thio(or oxy)]phenyl] alkyldiamide or alkylsulfonamide (7) [where Z is as described above and B is -NHCO- or $-NHSO_2-$]. The derivative (7) is then reacted with ammonia-saturated methanol at -5 to +5°C under an inert atmosphere or with sodium in methanol for several hours, giving N,N'-bis[4-[(α(or β)-D-glucopyranosyl)-thio(or oxy)]phenyl]alkyldiamide or alkylsulfonamide (8). Derivative (8) is then reacted with triethylamine-sulfur trioxide complex in N,N-dimethylacetamide, under an inert atmosphere at 60-65°C for several hours, giving the polytriethylammonium derivative (9) [where Y is $SO_3^{-}\cdot NH^{+}-(C_2H_5)_3$], which is then, if desired, reacted with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitated in ethanol, giving the end product (10) [where M is as described in the above Formula I].

FLOWCHART C

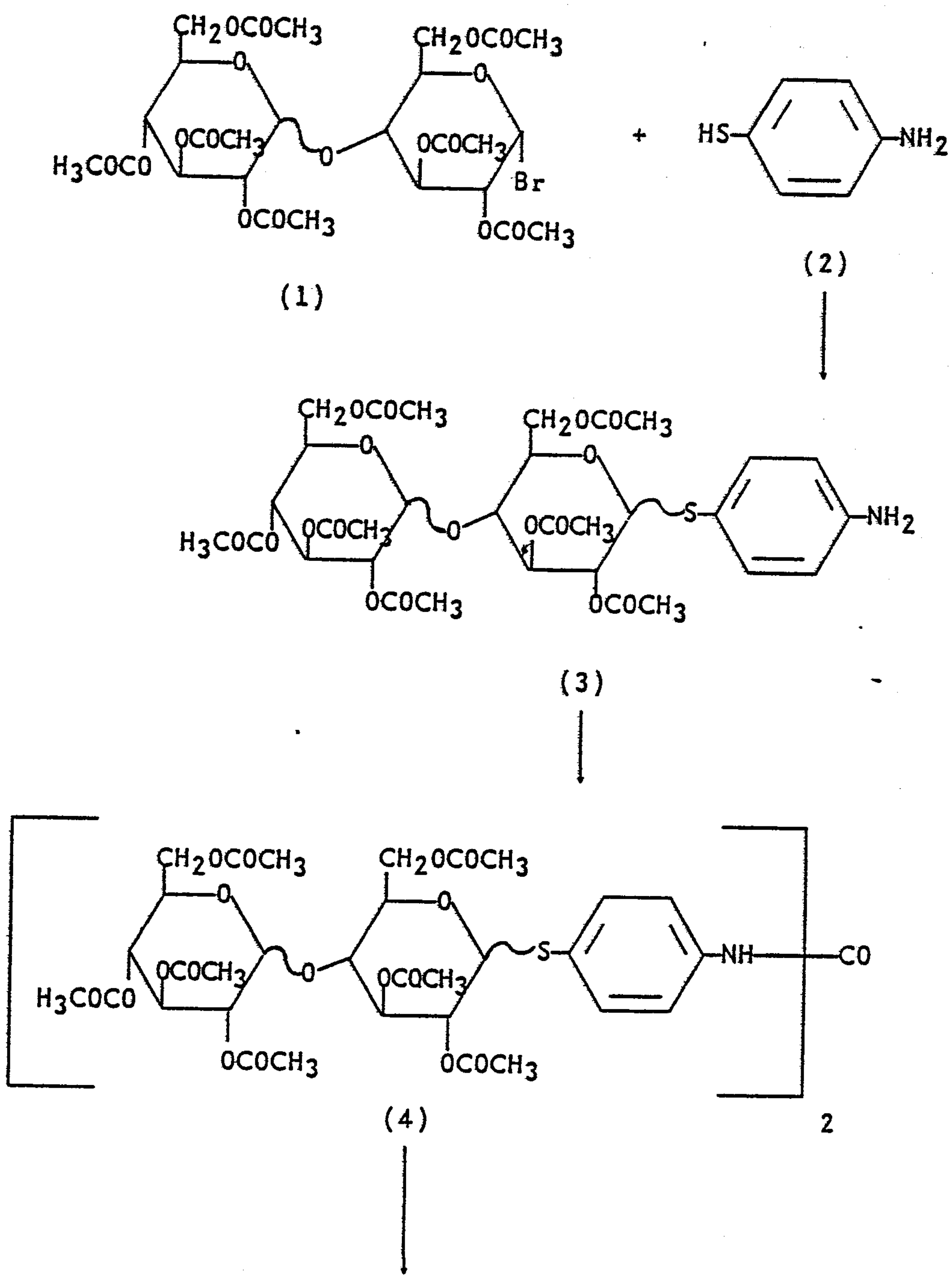

(1) (2) (3) (4)

FLOWCHART C (continued)

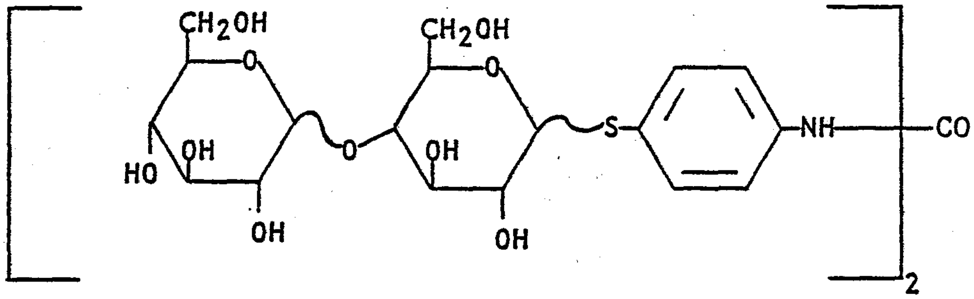

(5)

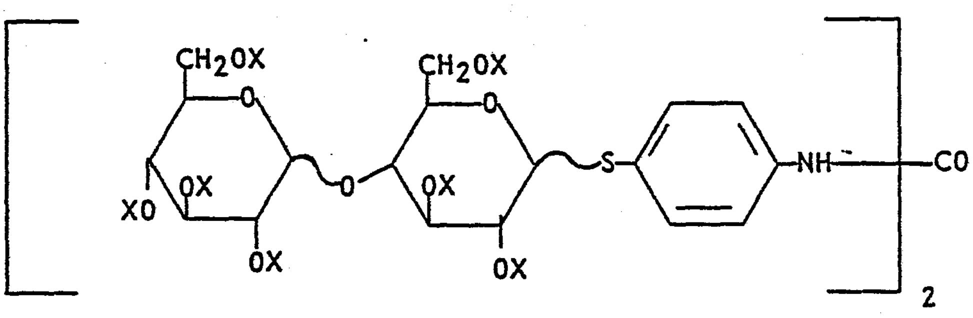

(6)

In accordance with the above Flowchart C, an acetobromomaltose (1) is reacted with 4-aminothiophenol (2) and sodium hydride in a solvent such as tetrahydrofuran at reflux for 10-20 hours, giving 4-aminophenyl 2,3,6-tri-O-acetyl-4-O-(2,3,4,6-tetra-O-acetyl-α(or β)-D-glucopyranosyl)-1-thio-α(or β)-D-glucopyranoside (3), which is then reacted with phosgene in a mixture of toluene and pyridine for 15-25 hours then purified by chromatography, giving N,N'-bis[4-[[2,3,6-tri-O-acetyl-4-O-(2,3,4,6-tetra-O-acetyl-α(or β)-D-glucopyranosyl)-α(or β)-D-glucopyranosyl]-thio]phenyl]urea (4), which is then reacted with methanol saturated with ammonia at -5 to +5°C, giving N,N'-bis-[4-[[4-O-(α(or β)-D-glucopyranosyl)-α(or β)-D-glucopyranosyl]thio]phenyl]urea (5), which is then reacted with triethylamine-sulfur trioxide in dry N,N-dimethylacetamide at 60-70°C for 40-60 hours giving (6), where X is $SO_3\overset{+}{N}H(C_2H_5)_3$, which is then, if desired, reacted with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitated in ethanol, giving the end product (6) of this invention.

FLOWCHART D

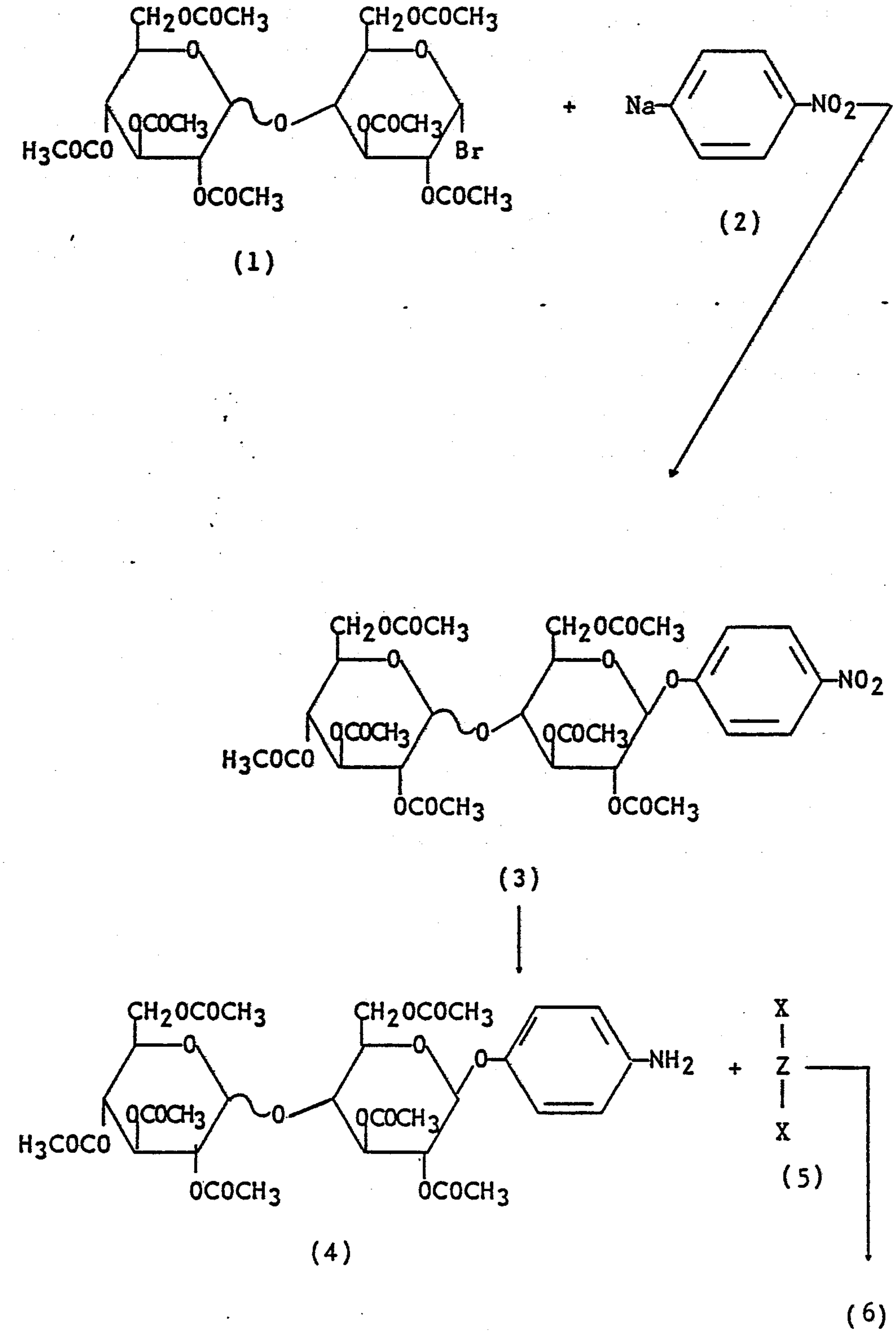

(1)

(2)

(3)

(4)

(5)

(6)

FLOWCHART D (continued)

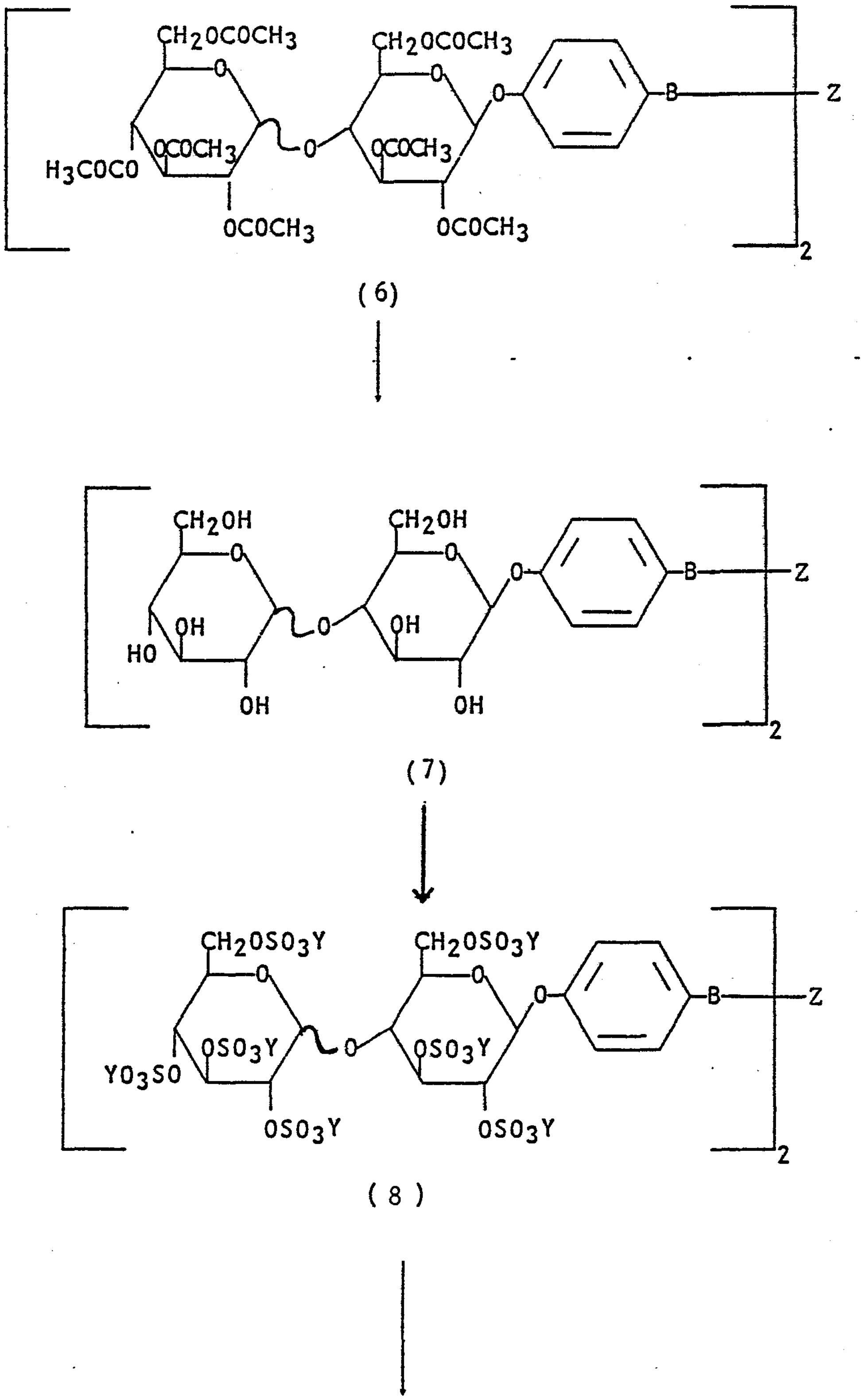

(6)

(7)

(8)

(9)

FLOWCHART D (continued)

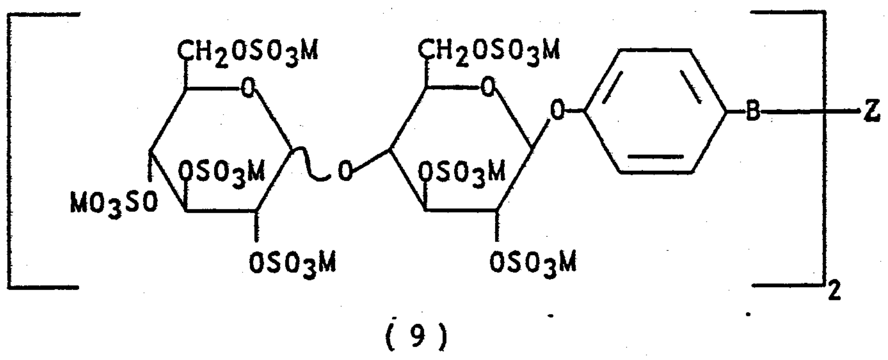

( 9 )

In accordance with the above Flowchart D, a brominated peracetyl sugar (1) is reacted with sodium p-nitrophenol (2), and potassium hydroxide in a solvent such as aqueous acetone for several hours, giving a 4-nitrophenyl 2,3,6-tri-O-acetyl-4-O-(2,3,4,6-tetra-O-acetyl-α(or β)-D-glucopyranosyl)-α(or β)-D-glucopyranoside (3) which is then catalytically reduced to the corresponding 4-aminophenyl derivative (4). The derivative (4) is then treated with an acid chloride (5) where X is, for example, COCl or $SO_2Cl$; Z is $-(CH_2)_m-$, and m is an integer 0-12 in a solvent such as acetonitrile under an inert atmosphere for several hours giving a N,N'-bis[4-[2,3,6-tri-O-acetyl-4-O-(2,3,4,6-tetra-O-acetyl-α(or β)-D-glucopyranosyl)-α(or β)-D-glucopyranosyloxy]phenyl]alkyldiamide (6), where Z is as described above and B is, for example, -NHCO-. The derivative (6) is then reacted with ammonia-saturated methanol at -5 to +5°C under an inert atmosphere or with sodium in methanol for several hours giving N,N'-bis[4-[4-O-(α(or β)-D-glucopyranosyl)-α(or β)-D-glucopyranosyloxy]phenyl]alkyldiamide (7) where B and Z are as described above. The derivative (6) may also be reduced with diborane in dry tetrahydrofuran under an inert atmosphere at -5 to +5°C, giving the derivative (7) where Z is as described above and B is $-NHCH_2-$. Derivative (7), where Z is as described above and B is -NHCO- or $-NHCH_2-$, is then reacted with triethylamine-sulfur trioxide complex in N,N-dimethylacetamide, under an inert atmosphere at 60-65°C for several hours, giving the tetradecatriethylammonium derivative (8), where Y is $\overset{+}{N}H(C_2H_5)_3$ which is then, if desired, reacted with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitated in ethanol, giving the end product (9) of this invention.

It is generally preferred that the respective product of each process step, described hereinabove, is separated and/or isolated prior to its use as starting material for subsequent steps. Separation and isolation can be effected by any suitable purification procedure such as, evaporation, crystallization, column chromatography, thin-layer chromatography, distillation, etc. Also it should be appreciated that when typical reaction conditions (e.g., temperatures, mole ratios, reaction times) have been given, the conditions which are both above and below these specified ranges can also be used, though generally less conveniently.

The term "pharmaceutically acceptable salts" refers to those salts of the parent compound which do not significantly or adversely affect the pharmaceutical properties (e.g., toxicity, effectiveness, etc.) of the parent compound. The salt forming moieties of the present invention which are pharmaceutically acceptable include the alkali metals (e.g., sodium, potassium, etc.); alkaline earth metals (e.g., calcium, etc.); aluminum; ammonia; and substituted ammonia selected from the group consisting of trialkylamine ($C_1$-$C_6$), piperidine, pyrazine, alkanolamine ($C_2$-$C_6$) and cycloalkylamine ($C_3$-$C_6$).

The term "trialkylamine ($C_1$-$C_6$)" defines those amines having three aliphatic fully saturated hydrocarbon substituents containing 1 to 6 carbon atoms either linearly or branched. Typically, these amines are trimethylamine, triethylamine, tripropylamine, dimethylethylamine, dimethyl-1-propylamine, etc. The term "alkanolamine ($C_2$-$C_6$)" refers to the above-defined trialkylamines additionally substituted with at least one and not more than three hydroxy groups on at least two of the alkyl hydrocarbon chains. Such amines are, for example, triethanolamine, tripropanolamine, etc. The term "cycloalkylamine ($C_3$-$C_6$)" is defined as the 3 to 6 fully saturated carbocyclic moieties such as cyclopropyl, methylcyclobutyl, cyclopentyl, cyclohexyl, etc.

The invention is illustrated by the following Examples. The preferred novel compounds of this invention are described in Examples 1-55. Pharmaceutical preparations are shown in Examples 56-73.

Example 1

4-Aminophenyl 2,3,6-tri-O-acetyl-4-O-(2,3,4,6-tetra-O-acetyl-α-D-glucopyranosyl)-1-thio-β-D-glucopyranoside

A solution of 5.13 g of 4-aminothiophenol in 50 ml of dry dimethoxyethane was added dropwise, at a rapid rate, to a stirred mixture of 1.8 g of 50% sodium hydride in oil, under argon. The mixture was stirred for 3-4 hours, then a solution of 25.8 g of acetobromo-α-D-maltose in 75 ml of dimethoxyethane was added dropwise, at a fast rate. The mixture was stirred for 18 hours, filtered and the filtrate concentrated to a brown oil. This oil was purified by dry column chromatography on 1300 g of silica gel, using ethyl acetate-hexane (1:1) as eluant. Crystallization of the residual oil from ether gave 13.8 g of the desired product as white crystals, m.p. 147-148°C.

Example 2

4-Nitrophenyl 2,3,6-tri-O-acetyl-4-O-(2,3,4,6-tetra-O-acetyl-α-D-glucopyranosyl)-1-thio-β-D-glucopyranoside

A 20.2 g sample of 50% sodium hydride in oil dispersion was washed free of oil with hexane and suspended in 300 ml of dry dimethoxyethane under argon. To the stirred suspension was added dropwise a solution of 65 g of 4-nitrothiophenol in 130 ml of dimethoxyethane. After the addition of 4-nitrothiophenol, stirring was continued for one hour, then a solution of 235 g of bromoheptaacetylmaltose in 800 ml of dry dimethoxyethane was added dropwise during one-half hour. After 2 hours of stirring at room temperature, excess sodium hydride was destroyed by the addition of 10 ml of acetic acid. Dimethoxyethane was evaporated under reduced pressure and the residue was partitioned between one liter

of methylene chloride and 500 ml of water. The layers were separated, the water layer was extracted once more with 500 ml of methylene chloride, the organic layers were combined, washed with brine, dried with magnesium sulfate, and the solution was passed through a pad of magnesium silicate. The pad was washed thoroughly with methylene chloride and ether, the solvents were evaporated, and the residual orange foam was crystallized from 2 liters of methanol to yield 179.5 g of faintly yellow crystals, m.p. 163-165°C.

Example 3

4-Aminophenyl 2,3,6-tri-O-acetyl-4-O-(2,3,4,6-tetra-O-acetyl-α-D-glucopyranosyl)-1-thio-β-D-glucopyranoside

To 91.3 g of 4-nitrophenyl 2,3,6-tri-O-acetyl-4-O-(2,3,4,6-tetra-O-acetyl-α-D-glucopyranosyl)-1-thio-β-D-glucopyranoside suspended in 200 ml of acetic acid was added 10 g of 10% palladium-on-charcoal catalyst in a Parr hydrogenation bottle, and this was hydrogenated on a Parr hydrogenator for 17 hours. The catalyst was filtered from the solution through diatomaceous earth and washed thoroughly with methanol. The solvents were evaporated *in vacuo*. The residue was dissolved in one liter of toluene and again evaporated. The residue was dissolved in methylene chloride and passed through a pad of magnesium silicate and the volume of the solution was adjusted to about 300 ml. To this was added 1.5 liters of ether and 5 g of activated charcoal, the solution was boiled for 3 minutes, the charcoal was filtered off and the solvents were again evaporated. The yellow foam was crystallized from 1.5 liters of ether to yield 69.6 g of light yellow crystals, isolated in three crops, m.p. 145-148°C.

Example 4

N,N'-Bis[4-[2,3,6-tri-O-acetyl-4-O-(2,3,4,6-tetra-O-acetyl-α-D-glucopyranosyl)-β-D-glucopyranosylthio]phenyl]octanediamide

To a stirred solution of 25 g of 4-aminophenyl 2,3,6-tri-O-acetyl-4-O-(2,3,4,6-tetra-O-acetyl-α-D-glucopyranosyl)-1-thio-β-D-glucopyranoside in 250 ml of a dry mixture of pyridine-acetonitrile (1:1) was added a solution of 3.55 g of suberoyl chloride in 10 ml of acetonitrile. This mixture was allowed to stand, under argon, for 18 hours. An additional 0.2 ml of suberoyl chloride was added, the solution was allowed to stand for an additional 18 hours and was then poured into 2 liters of water. After stirring for 15 minutes, the oil was separated and extracted into 500 ml of dichloromethane. The extract was washed twice with 500 ml portions of 0.5N hydrochloric acid, then with saturated aqueous sodium chloride solution, dried and taken to dryness, giving 28 g of the desired product as a white foam.

Examples 5-13

Treatment of 4-aminophenyl 2,3,6-tri-O-acetyl-4-O-(2,3,4,6-tetra-O-acetyl-α-D-glucopyranosyl)-1-thio-β-D-glucopyranoside or other appropriate disaccharides, as well as the -1-oxy- derivatives of this and other appropriate disaccharides, with other acid chlorides according to the procedure of Example 4, gave the products of Examples 5-13 as shown in the following Table I.

TABLE I

| Example | Acid Chloride | Product |
|---|---|---|
| 5 | Succinyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]butanediamide |
| 6 | Glutaryl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]pentanediamide |
| 7 | Adipoyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]hexanediamide |
| 8 | Pimeloyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]heptanediamide |
| 9 | Sebacyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]decanediamide |
| 10 | Malonyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]propanediamide |

TABLE I (continued)

| Example | Acid Chloride | Product |
|---|---|---|
| 11 | Tetradecanedioyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]tetradecanediamide |
| 12 | Oxalyl chloride | N,N'-Bis[4-[2,3,6-tri-O-acetyl-4-O-(2,3,4,6-tetra-O-acetyl-α-D-glucopyranosyl)-β-D-glucopyranosylthio]phenyl]ethanediamide |
| 13 | Glutaryl chloride | N,N'-Bis[4-[2,3,6-tri-O-acetyl-4-O-(2,3,4,6-tetra-O-acetyl-β-D-glucopyranosyl)-β-D-glucopyranosylthio]phenyl]pentanediamide |

Example 14

N,N'-Bis[4-[4-O-(α-D-glucopyranosyl)-β-D-glucopyranosylthio]phenyl]octanediamide

A 28.4 g portion of N,N'-bis[4-[2,3,6-tri-O-acetyl-4-O-(2,3,4,6-tetra-O-acetyl-α-D-glucopyranosyl)-β-D-glucopyranosylthio]phenyl]octanediamide was dissolved in a 0°C solution of 500 ml of methanol saturated with ammonia. The solution was allowed to stand at 0°C, under an inert atmosphere, for 18 hours, then was warmed to ambient temperature and evaporated. The residue was triturated with 200 ml of absolute ethanol, filtered, washed in sequence with absolute ethanol, acetonitrile, then ether and dried *in vacuo* at 40°C, giving 12.1 g of the desired intermediate as an amorphous solid.

Examples 15-23

Treatment of the products of Examples 5-13 by the procedure of Example 14 resulted in the intermediate products of Examples 15-23, given in Table II.

TABLE II

| Example | Starting Material (Example) | Product |
|---|---|---|
| 15 | 5 | N,N'-Bis[4-[4-O-(α-D-glucopyranosyl)-β-D-glucopyranosylthio]phenyl]butanediamide |
| 16 | 6 | N,N'-Bis[4-[4-O-(α-D-glucopyranosyl)-β-D-glucopyranosylthio]phenyl]pentanediamide |
| 17 | 7 | N,N'-Bis[4-[4-O-(α-D-glucopyranosyl)-β-D-glucopyranosylthio]phenyl]hexanediamide |
| 18 | 8 | N,N'-Bis[4-[4-O-(α-D-glucopyranosyl)-β-D-glucopyranosylthio]phenyl]heptanediamide |
| 19 | 9 | N,N'-Bis[4-[4-O-(α-D-glucopyranosyl)-β-D-glucopyranosylthio]phenyl]decanediamide |
| 20 | 10 | N,N'-Bis[4-[4-O-(α-D-glucopyranosyl)-β-D-glucopyranosylthio]phenyl]propanediamide |
| 21 | 11 | N,N'-Bis[4-[4-O-(α-D-glucopyranosyl)-β-D-glucopyranosylthio]phenyl]tetradecanediamide |

TABLE II (continued)

| Example | Starting Material (Example) | Product |
|---|---|---|
| 22 | 12 | N,N'-Bis[4[4-O-(α-D-glucopyranosyl)-β-D-glucopyranosylthio]phenyl]ethanediamide |
| 23 | 13 | N,N'-Bis[4-[4-O-(β-D-glucopyranosyl)-β-D-glucopyranosylthio]phenyl]pentanediamide |

Example 24

N,N'-Bis[4-[4-O-(α-D-glucopyranosyl)-β-D-glucopyranosylthio]phenyl]butanediamine

A 9.2 g portion of N,N'-bis[4-[2,3,6-tri-O-acetyl-4-O-(2,3,4,6-tetra-O-acetyl-α-D-glucopyranosyl)-β-D-glucopyranosylthio]phenyl]butanediamide in 30 ml of dry tetrahydrofuran was cooled in an ice bath. To this was added with magnetic stirring dropwise 210 ml of 1M solution of diborane in tetrahydrofuran. The solution was allowed to come to room temperature overnight and the excess diborane was decomposed by the addition of methanol. The white solid which separated during the reaction was collected by filtration and was suspended in 500 ml of methanol saturated with ammonia at 0°C. The mixture was stirred overnight and the solid gradually dissolved as the temperature rose to room temperature. After evaporation of the solution, the residue was triturated in refluxing ethanol for one hour. The solid was collected by filtration and dried. There was obtained 2.51 g as an off-white solid, $[\alpha]_D^{26°} = 25° \pm 1 (H_2O)$.

Example 25

Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]octanediamide

Into a warm solution of 91 g of triethylamine sulfur trioxide complex and 91 g of 4A molecular sieves in dry N,N-dimethylacetamide was dissolved 14.9 g of N,N'-bis-[4-[4-O-(α-D-glucopyranosyl)-β-D-glucopyranosylthio]phenyl]octanediamide. The resulting solution was allowed to stand at 60-65°C in an oil bath, under argon, for 66 hours. The mixture was filtered and the filtrate poured into 3500 ml of acetone. After standing for one hour, the supernatant solution was decanted and the residual gum (tetradecatriethylammonium salt) was washed with three 150 ml portions of acetone. The gum was then dissolved in a solution

composed of 19.7 g of anhydrous sodium acetate in 100 ml of water. This solution was added in a thin stream, with stirring, to 3500 ml of absolute ethanol, stirred for 20 minutes and then allowed to stand for 2 hours. The supernatant was siphoned from the solid which was then collected on a filter and washed repeatedly with absolute ethanol and finally with ether. Vacuum drying at 40°C provided 33.6 g of the desired product as an amorphous powder.

To remove residual amounts of sodium sulfate the product was redissolved in 200 ml of water and mixed with 40 ml of 1M barium acetate solution. The solution was centrifuged at 3000 rpm for 30 minutes and the cloudy supernatant was decanted and filtered through a 90S clarification filter, which in turn was washed with a small amount of water. The clear filtrate was passed through 400 g of activated Amberlite® CG-120 ion exchange resin ($Na^+$ form), packed into a 60 mm. ID glass column. The sample was washed off the column with distilled water and fractions containing carbohydrate were pooled. The combined fractions were evaporated to 150 ml and the title compound was precipitated with 4 liters of hot ethanol. The solid was collected and dried in vacuo at 110°C giving 23.9 g of yellow powder, $[\alpha]_D^{26°} = +17° \pm 1$ (1.1%, $H_2O$).

Examples 26-35

Following the procedure of Example 25, the intermediates of Examples 15-24 are converted to the products of Examples 26-35 as given in Table III.

TABLE III

| Example | Intermediate (Example) | Product |
|---|---|---|
| 26 | 15 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]butanediamide |
| 27 | 16 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]pentanediamide |
| 28 | 17 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]hexanediamide |
| 29 | 18 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]heptanediamide |
| 30 | 19 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]decanediamide |
| 31 | 20 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]propanediamide |

TABLE III(continued)

| Example | Intermediate (Example) | Product |
|---|---|---|
| 32 | 21 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]tetradecanediamide |
| 33 | 22 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]ethanediamide |
| 34 | 23 | Tetradecasodium N,N'-bis[4-[2,3,6-tri-O-sulfo-4-O-(2,3,4,6-tetra-O-sulfo-β-D-glucopyranosyl)-β-D-glucopyranosylthio]phenyl]pentanediamide |
| 35 | 24 | Tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]butanediamine |

## Example 36

### 4-Nitrophenyl 2,3,6-tri-O-acetyl-4-O-(2,3,4,6-tetra-O-acetyl-α-D-glucopyranosyl)-β-D-glucopyranoside

A mixture of 50 g of sodium 4-nitrophenol, 100 g of bromoheptaacetylmaltose, 1.0 g of potassium hydroxide, 60 ml of water, and 100 ml of acetone was stirred at ambient temperature for 20 hours. The reaction was diluted with 2 l of water, the resultant oil was allowed to settle, and the aqueous layer was decanted. The oil was dissolved into 500 ml of methylene chloride, washed with saturated sodium bicarbonate solution, dried, and passed through a pad of hydrous magnesium silicate. Evaporation of the solvent left a light yellow oil which was crystallized from 450 ml methanol to yield 27.5 g of white crystals, m.p. 165-170°C.

## Example 37

### 4-Aminophenyl 2,3,6-tri-O-acetyl-4-O-(2,3,4,6-tetra-O-acetyl-α-D-glucopyranosyl)-β-D-glucopyranoside

To 27.4 g of 4-nitrophenyl 2,3,6-tri-O-acetyl-4-O-(2,3,4,6-tetra-O-acetyl-α-D-glucopyranosyl)-β-D-glucopyranoside suspended in 150 ml of acetic acid was added 2.7 g of 10% palladium-on-charcoal catalyst in a Parr hydrogenation bottle, and this was hydrogenated on a Parr hydrogenator for 17 hours. The catalyst was filtered from the solution through diatomaceous earth and washed thoroughly with methanol. The solvents were evaporated *in vacuo*. The residue was dissolved in one liter of toluene and again evaporated. The residue was dissolved in methylene chloride and passed through a pad of magnesium silicate and the solvent was evaporated to yield the title compound as a light yellow oil. A 3.5 g portion of this was crystallized from methanol to yield 2.6 g of an off-white crystal, m.p. 173-175°C.

Example 38

N,N'-Bis[4-[2,3,6-tri-O-acetyl-4-O-(2,3,4,6-tetra-O-acetyl-α-D-glucopyranosyl)-β-D-glucopyranosyloxy]phenyl]butanediamide

To a stirred solution of 10 g of 4-aminophenyl 2,3,6-tri-O-acetyl-4-O-(2,3,4,6-tetra-O-acetyl-α-D-glucopyranosyl)-β-D-glucopyranoside in 100 ml of a dry mixture of pyridine-acetonitrile (1:1) under argon was added a solution of 1.14 g of succinyl chloride in 10 ml of acetonitrile. This mixture was allowed to stir, under argon, for 18 hours. The solution was poured into 900 ml of water and stirred for 15 minutes. The oil was then separated and extracted into 500 ml of ethyl acetate and passed through a pad of hydrous magnesium silicate. Evaporation yielded 7.25 g of the title compound, which was chromatographed on 800 g of silica gel and eluted with ethyl acetate/hexane. Fractions containing the desired product were combined and evaporated to 6.4 g of a white foam, $[\alpha]_D^{26^o} = +45^o \pm 2$ (0.64%, $CHCl_3$).

Example 39

N,N'-Bis[4-[4-O-(α-D-glucopyranosyl)-β-D-glucopyranosyloxy]phenyl]butanediamide

A 3.0 g portion of N,N'-bis[4-[2,3,6-tri-O-acetyl-4-O-(2,3,4,6-tetra-O-acetyl-α-D-glucopyranosyl)-β-D-glucopyranosyloxy]phenyl]butanediamide was dissolved in a 0°C solution of 50 ml of methanol saturated with ammonia. The solution was allowed to stand at room temperature, under an inert atmosphere, for 18 hours, then was evaporated. The residue was crystallized from methanol/water to yield 1.35 g of the title compound as a dihydrate, m.p. 190-200°C.

Example 40

N,N'-Bis[4-[4-O-(α-D-glucopyranosyl)-β-D-glucopyranosyloxy]phenyl]butanediamine

A 3.79 g portion of N,N'-bis[4-[2,3,6-tri-O-acetyl-4-O-(2,3,4,6-tetra-O-acetyl-α-D-glucopyranosyl)-β-D-glucopyranosyloxy]phenyl]butanediamide was added to 25 ml

of tetrahydrofuran. This mixture was flushed with argon and cooled in an ice-water bath. A 100 ml portion of 1M diborane in tetrahydrofuran was added at a rapid rate with stirring and upon completion, the cooling bath was removed. The reaction was stirred at ambient temperature overnight, then refluxed for 1/2 hour, cooled and the diborane decomposed by the addition of 100 ml of methanol. The resulting solid was collected, giving 640 mg of the desired intermediate as a white powder, m.p. 285°C (dec.).

Example 41

Tetradecasodium N,N'-bis[4-[2,3,6-tri-O-sulfo-4-O-(2,3,4,6-tetra-O-sulfo-α-D-glucopyranosyl)-β-D-glucopyranosyloxy]phenyl]butanediamide

Into a warm solution of 5.35 g of triethylamine-sulfur trioxide complex and 4 g of 4A molecular sieves in 20 ml dry N,N-dimethylacetamide was dissolved 1.0 g of N,N'-bis-4-[4-O-(α-D-glucopyranosyl-β-D-glucopyranosyloxy]-phenyl]butanediamide. The resulting solution was allowed to stand at 60-65°C in an oil bath, under argon, for 17 hours. The mixture was filtered and the filtrate poured into 400 ml of acetone. After standing for one hour, the supernatant solution was decanted and the residual gum (tetradecatriethylammonium salt) was washed with three 50 ml portions of acetone. The gum was then dissolved in a solution composed of 2 g of anhydrous sodium acetate in 10 ml of water. This solution was added in a thin stream, with stirring, to 500 ml of absolute ethanol, stirred for 20 minutes and then allowed to stand for 2 hours. The supernatant was siphoned from the solid which was then collected on a filter and washed repeatedly with absolute ethanol and finally with ether. Vacuum drying at 40°C provided 3.0 g of the desired product as an amorphous powder.

To remove residual amounts of sodium sulfate the product was redissolved in 50 ml of water and mixed with 5 ml of 1M barium acetate solution. The solution was centrifuged at 3000 rpm for 30 minutes and the cloudy supernatant was decanted and filtered through a 90S clarification filter, which in turn is washed with a small amount of water. The clear filtrate is passed through 100 g of activated Amberlite® CG-120 ion exchange resin ($Na^+$ form), packed into a 30 mm ID glass column. The sample was washed off the column with distilled water and fractions containing carbohydrate were pooled. The combined fractions are evaporated to 15 ml and the title compound was precipitated with 500 ml of hot ethanol. The solid was collected and dried in vacuo giving 2.3 g of white powder, $[\alpha]_D^{26°}$ +14°± 5 (0.27%, $H_2O$).

Example 42

Following the procedure of Example 7, N,N'-bis-[4-[2,3,6-tri-O-sulfo-4-O-(2,3,4,6-tetra-O-sulfo-α-D-glucopyranosyl)-β-D-glucopyranosyloxy]phenyl]butanediamine is converted to the corresponding tetradecasodium salt.

Example 43

N,N'-Bis[4-[(2,3,4,6-tetra-O-acetyl-β-D-glucopyranosyl)thio]phenyl]pentanediamide

To a mixture of 3.0 g of 50% sodium hydride in oil dispersion in 40 ml of dry dimethoxyethane under argon was added dropwise a solution of 8.7 g of 4-aminothiophenol in 70 ml of dry dimethoxyethane. The mixture was stirred for 3.5 hours, then a solution of 25.5 g of α-D-bromoglucose tetraacetate in 70 ml of dry dimethoxyethane was added dropwise at a fast rate. The mixture was stirred overnight, filtered through diatomaceous earth and the filtrate evaporated to a glass. This glass was dissolved in 150 ml of dichloromethane, filtered through hydrous magnesium silicate, washed with one liter of dichloromethane, treated with charcoal and filtered through diatomaceous earth. The filtrate was evaporated to a glass which was dissolved in 150 ml of hot ether and then refrigerated overnight, giving 15.8 g of 4-aminophenylthio 2,3,4,6-tetra-O-acetyl-β-D--glucopyranoside.

To a solution of 5.0 g of the above compound in

50 ml of dry pyridine:acetonitrile (1:1) stirring under argon, was added dropwise a solution of 930 mg of glutaryl chloride in 3 ml of acetonitrile. This mixture was stirred for 2 hours, a small portion of glutaryl chloride was added, the mixture was allowed to stand overnight, then poured into 250 ml of water and stirred for 15 minutes. The resulting gum was extracted twice with chloroform, the extracts were combined, washed with 0.5% hydrochloric acid, dried, treated with charcoal and filtered through diatomaceous earth. The filtrate was evaporated to a glass which was dissolved in 100 ml of chloroform, filtered through hydrous magnesium silicate, washed with 600 ml of chloroform and evaporated, giving the desired intermediate as 4 g of a tan glass.

Example 44

N,N'-Bis[4-[(β-D-glucopyranosyl)thio]phenyl]-pentanediamide

To 4.0 g of N,N'-bis[4-[(2,3,4,6-tetra-O-acetyl-β-D-glucopyranosyl)thio]phenyl]pentanediamide was added 500 ml of ammonia saturated methanol at 0°C. The solution was kept at 0°C for 2 hours, then at ambient temperature overnight and taken to dryness *in vacuo*. The residue was triturated with 20 ml of absolute ethanol and the resulting solid was collected, giving 1.45 g of the desired intermediate as an amorphous solid.

Example 45

Octasodium N,N'-bis[4-[(2,3,4,6-tetra-O-sulfo-β-D-glucopyranosyl)thio]phenyl]pentanediamide

A 6.75 g portion of triethylamine-sulfur trioxide complex was dissolved in 30 ml of dry N,N-dimethylacetamide. A 6.75 g portion of 4A molecular sieves was added and the mixture was heated at 60°C for 15 minutes. A 1.25 g portion of N,N'-bis[4-[(β-D-glucopyranosyl)thio]phenyl]-pentanediamide was added and the mixture was heated at 60°C under argon for 4 hours, then cooled overnight, poured into 650 ml of acetone and refrigerated overnight. The

resulting gum (octatriethylammonium N,N'-bis[4-[(2,3,4,6-tetra-O-sulfo-β-D-glucopyranosyl)thio]phenyl]pentanediamide) was separated, washed with acetone and added to 10 ml of water containing 1.5 g of sodium acetate. The mixture was filtered through diatomaceous earth and the filtrate added to 600 ml of absolute ethanol, stirred for 30 minutes and allowed to stand overnight. The solid was collected, washed with absolute ethanol, then ether and dried *in vacuo*, giving 1.57 g of the desired product.

## Example 46

## (1,4-Dioxo-1,4-butanediyl)bis(imino-4,1-phenylene)-bis(2,3,4-tri-O-acetyl-1-thio-β-D-glucopyranosiduronic acid), dimethyl ester

To a mixture of 700 mg of 50% sodium hydride oil dispersion in 50 ml of dry dimethoxyethane, stirred under argon was added dropwise a solution of 3.0 g of sodium 4-aminothiophenol in 25 ml of dry dimethoxyethane. The mixture was stirred for 2 hours, then a solution of 4.8 g of methyl acetobromoglucuronate in 25 ml of dry dimethoxyethane was added. The mixture was stirred overnight, filtered through diatomaceous earth, washed with dimethoxyethane and taken to dryness. The residue was taken up in chloroform, filtered through hydrous magnesium silicate, washed with one liter of chloroform and taken to dryness, giving a syrup. This syrup was chromatographed on 600 g of silica gel in a nylon column, the column being divided into 36 equal segments. Segments 15-23 were combined, suspended in ethyl acetate, filtered free of silica gel, and taken to dryness *in vacuo*, giving a yellow glass which was crystallized from ether, giving 4-aminophenylthio 2,3,4-tri-O-acetyl-β-D-glucopyranosiduronic acid, methyl ester as off-white crystals.

To a stirred solution of 2.0 g of the above compound in 20 ml of pyridine:acetonitrile (1:1) under argon was added dropwise a solution of 365 mg of succinyl chloride in 3 ml of acetonitrile. The mixture was allowed to stand 4

hours and then added with stirring to 125 ml of water. The mixture was stirred 15 minutes, then the solid was collected, dried, dissolved in 50 ml of chloroform and filtered through hydrous magnesium silicate, washing with 600 ml of chloroform. The filtrate was evaporated to a white glass. The addition of methanol and chilling gave crystals which were washed with cold methanol, then ether and dried, giving 785 mg of the desired intermediate as white crystals, m.p. 220°C.

## Example 47

**(1,4-Dioxo-1,4-butanediyl)bis(imino-4,1-phenylene)-bis(1-thio-β-D-glucopyranosiduronic acid), dimethyl ester**

(1,4-Dioxo-1,4-butanediyl)bis(imino-4,1-phenylene)bis(2,3,4-tri-O-acetyl-1-thio-β-D-glucopyranosiduronic acid), dimethyl ester may be converted to the desired intermediate essentially by the procedure of Example 2.

## Example 48

**Hexasodium (1,4-dioxo-1,4-butanediyl)bis(imino-4,1-phenylene)bis(2,3,4-tri-O-sulfo-1-thio-β-D-glucopyranosiduronic acid), dimethyl ester**

(1,4-Dioxo-1,4-butanediyl)bis(imino-4,1-phenylene)bis(1-thio-β-D-glucopyranosiduronic acid), dimethyl ester may be converted to the desired product essentially by the procedure of Example 3.

## Example 49

**(1,5-Dioxo-1,5-pentanediyl)bis(imino-4,1-phenylene)-bis(2,3,4-tri-O-acetyl-1-thio-β-D-glucopyranosiduronic acid), dimethyl ester**

To a solution of 2.0 g of 4-aminophenylthio 2,3,4-tri-O-acetyl-β-D-glucopyranosiduronic acid, methyl ester in 20 ml of pyridine:acetonitrile (1:1) under argon, with swirling, was added a solution of 383 mg of glutaryl chloride in 3 ml of acetonitrile. The procedure of Example 1 was followed, giving 1.8 g of the desired intermediate as a cream colored foam.

Example 50

(1,5-Dioxo-1,5-pentanediyl)bis(imino-4,1-phenylene)-bis(1-thio-β-D-glucopyranosiduronic acid)

To a solution of 1.7 g of (1,5-dioxo-1,5-pentanediyl)bis(imino-4,1-phenylene)bis(2,3,4-tri-O-acetyl--1-thio-β-D-glucopyranosiduronic acid), dimethyl ester in 30 ml of methanol was added 1.74 ml of 5N sodium hydroxide with stirring, under an argon atmosphere. Stirring was continued for one hour, then 2.0 ml of 6N glacial acetic acid was added and the solution was taken to dryness. The residue was evaporated three times from toluene, giving a glass which was triturated with 150 ml of absolute ethanol, giving 1.29 g of the desired intermediate as an amorphous solid.

Example 51

Octasodium (1,5-dioxo-1,5-pentanediyl)bis-(imino-4,1-phenylene)bis(2,3,4-tri-O-sulfo-1-thio-β-D-glucopyranosiduronic acid)

A 5.18 g portion of triethylamine-sulfur trioxide complex was reacted with 1.0 g of (1,5-dioxo-1,5-pentanediyl)bis(imino-4,1-phenylene)bis(1-thio-β-D-glucopyranosiduronic acid) and then with sodium acetate as described in Example 3, giving 1.13 g of the desired product as a cream colored amorphous solid.

Example 52

4-Aminophenyl 2,3,6-tri-O-acetyl-4-O-(2,3,4,6-tetra-O-acetyl-α-D-glucopyranosyl)-1-thio-β-D-glucopyranoside

To a slurry of 0.6 g of hexane-washed sodium hydride (50% oil dispersion) in 50 ml of dry dimethoxyethane was added a solution of 1.7 g of 4-aminothiophenol in 25 ml of dry dimethoxyethane. The mixture was stirred for 1/2 hour, then 8.7 g of acetobromomaltose in 20 ml. of dimethoxyethane was added. This solution was stirred overnight, then refluxed for 7 hours. The reaction was filtered and the filtrate was extracted with 50 ml of 10% sodium carbonate and 50 ml of water. The organic layer

was dried, concentrated to a solid which was purified by chromatography, giving 4.1 g of the desired compound $[\alpha]_D^{26}$ = +44° ($CHCl_3$).

Example 53

N,N'-Bis[4-[[2,3,6-tri-O-acetyl-4-O-(2,3,4,6-tetra-O-acetyl-α-D-glucopyranosyl)-β-D-glucopyranosyl]thio]phenyl]urea

To a solution of 21.4 g of 4-aminophenyl 2,3,6-tri-O-acetyl-4-O-(2,3,4,6-tetra-O-acetyl-α-D-glucopyranosyl)-1-thio-β-D-glucopyranoside in 200 ml of toluene and 25 ml of pyridine there was added 5.53 ml of toluene containing 1.77 g of phosgene. This mixture was stirred for 18 hours, then concentrated, dissolved in dichloromethane, washed with water, then saline, dried and reconcentrated. This concentrate was purified by high performance liquid chromatography, giving 10.3 g of the desired intermediate.

Example 54

N,N'-Bis[4-[[4-O-(α-D-glucopyranosyl)-β-D-glucopyranosyl]thio]phenyl]urea

A mixture of 17.3 g of N,N'-bis[4-[[2,3,6-tri-O-acetyl-4-O-(2,3,4,6-tetra-O-acetyl-α-D-glucopyranosyl)-β-D-glucopyranosyl]thio]phenyl]urea in 500 ml of methanol saturated with ammonia was stirred in an ice bath for 6 hours and then refrigerated overnight. The mixture was filtered through a sintered glass funnel and concentrated *in vacuo* to a foam. The foam was triturated with ethanol, filtered, washed with ethanol then ether and dried, giving 11.42 g of the desired intermediate.

Example 55

Tetradecasodium N,N'-bis[4-[[2,3,6-tri-O-sulfo-4-O-(2,3,4,6-tetra-O-sulfo-α-D-glucopyranosyl)-β-D-glucopyranosyl]thio]phenyl]urea

A 72.0 g portion of triethylamine-sulfur trioxide complex was dissolved in 350 ml. of dry N,N-dimethylacetamide containing 72.0 g of 4A molecular sieves. This mixture was heated at 60-65°C for 20 minutes. A 10.5 g portion of N,N'-bis[4-[[4-O-(α-D-glucopyranosyl)-β-D-glucopyranosyl]thio]phenyl]urea was added and the mixture was heated at 63-65°C for 48 hours. The mixture was then cooled, filtered and the filtrate added to 4 liters of acetone and stirred giving a solid which is the tetradecatriethylammonium derivative of the title product.

This solid was dissolved in 85 ml of water containing 15.6 g of sodium acetate, stirred for 20 minutes, filtered and the filtrate slowly added to 4 liters of absolute ethanol and stirred for 1/2 hour. The resulting solid was collected, washed with ethanol and dried giving 16.22 g of the desired product as a light tan solid.

Example 56

Preparation of Compressed Tablet

| Ingredient | mg./Tablet |
|---|---|
| Active Compound......................... | 0.5-500 |
| Dibasic Calcium Phosphate N.F. ......... | qs |
| Starch USP.............................. | 40 |
| Modified Starch......................... | 10 |
| Magnesium Stearate USP.................. | 1-5 |

Example 57

Preparation of Compressed Tablet - Sustained Action

| Ingredient | mg./Tablet |
|---|---|
| Active Compound as Aluminum............ Lake*, Micronized | 0.5-500 (as acid equivalent) |
| Dibasic Calcium Phsophate N.F. ............... | qs |
| Alginic Acid................................. | 20 |
| Starch USP................................... | 35 |
| Magnesium Stearate USP....................... | 1-10 |

*Complement inhibitor plus aluminum sulfate yields aluminum complement inhibitor. Complement inhibitor content in aluminum lake ranges from 5-30%.

Example 58

Preparation of Hard Shell Capsule

| Ingredient | mg./Capsule |
| --- | --- |
| Active Compound | 0.5-500 |
| Lactose, Spray Dried | qs |
| Magnesium Stearate | 1-10 |

Example 59

Preparation of Oral Liquid (Syrup)

| Ingredient | % W/V |
| --- | --- |
| Active Compound | 0.05-5 |
| Liquid Sugar | 75.0 |
| Methyl Paraben USP | 0.18 |
| Propyl Paraben USP | 0.02 |
| Flavoring Agent | qs |
| Purified Water qs ad | 100.0 |

Example 60

Preparation of Oral Liquid (Elixir)

| Ingredient | % W/V |
| --- | --- |
| Active Compound | 0.05-5 |
| Alcohol USP | 12.5 |
| Glycerin USP | 45.0 |
| Syrup USP | 20.0 |
| Flavoring Agent | qs |
| Purified Water qs ad | 100.0 |

Example 61

Preparation of Oral Suspension (Syrup)

| Ingredient | % W/V |
| --- | --- |
| Active Compound as Aluminum Lake, Micronized | 0.05-5 (acid equivalent) |
| Polysorbate 80 USP | 0.1 |
| Magnesium Aluminum Silicate, Colloidal | 0.3 |
| Flavoring Agent | qs |
| Methyl Paraben USP | 0.18 |
| Propyl Paraben USP | 0.02 |
| Liquid Sugar | 75.0 |
| Purified Water qs ad | 100.0 |

Example 62

Preparation of Injectable Solution

| Ingredient | % W/V |
|---|---|
| Active Compound............. | 0.05-5 |
| Benzyl Alcohol N.F. ........ | 0.9 |
| Water for Injection......... | 100.0 |

Example 63

Preparation of Injectable Oil

| Ingredient | % W/V |
|---|---|
| Active Compound........... | 0.05-5 |
| Benzyl Alcohol............ | 1.5 |
| Sesame Oil qs ad.......... | 100.0 |

Example 64

Preparation of Intra-Articular Product

| Ingredient | Amount |
|---|---|
| Active Compound..................... | 2-20 mg. |
| NaCl (physiological saline).......... | 0.9% |
| Benzyl Alcohol....................... | 0.9% |
| Sodium Carboxymethylcellulose........ | 1-5% |
| pH adjusted to 5.0-7.5 | |
| Water for Injection qs ad............ | 100% |

Example 65

Preparation of Injectable Depo Suspension

| Ingredient | % W/V |
|---|---|
| Active Compound...................... | 0.05-5 (acid equivalent) |
| Polysorbate 80 USP................... | 0.2 |
| Polyethylene Glycol 4000 USP......... | 3.0 |
| Sodium Chloride USP.................. | 0.8 |
| Benzyl Alcohol N.F. ................. | 0.9 |
| HCl to pH 6-8........................ | qs |
| Water for Injection qs ad............ | 100.0 |

## Example 66

### Preparation of Dental Paste

| Ingredient | % W/V |
|---|---|
| Active Compound | 0.05-5 |
| Zinc Oxide | 15 |
| Polyethylene Glycol 4000 USP | 50 |
| Distilled Water qs. | 100 |

## Example 67

### Preparation of Dental Ointment

| Ingredient | % W/W |
|---|---|
| Active Compound | 0.05-5 |
| Petrolatum, White USP qs | 100 |

## Example 68

### Preparation of Dental Cream

| Ingredient | % W/W |
|---|---|
| Active Compound | 0.05-5 |
| Mineral Oil | 50 |
| Beeswax | 15 |
| Sorbitan Monostearate | 2 |
| Polyoxyethylene 20 Sorbitan Monostearate | 3 |
| Methyl Paraben USP | 0.18 |
| Propyl Paraben USP | 0.02 |
| Distilled Water qs | 100 |

## Example 69

### Preparation of Topical Cream

| Ingredient | % W/W |
|---|---|
| Active Compound | 0.05-5 |
| Sodium Lauryl Sulfate | 1 |
| Propylene Glycol | 12 |
| Stearyl Alcohol | 25 |
| Petrolatum, White USP | 25 |
| Methyl Paraben USP | 0.18 |
| Propyl Paraben USP | 0.02 |
| Purified Water qs | 100 |

Example 70

Preparation of Topical Ointment

| Ingredient | % W/W |
|---|---|
| Active Compound.................... | 0.05-5 |
| Cholesterol........................ | 3 |
| Stearyl Alcohol.................... | 3 |
| White Wax.......................... | 8 |
| Petrolatum, White USP qs........... | 100 |

Example 71

Preparation of Spray Lotion (Non-aerosol)

| Ingredient | % W/W |
|---|---|
| Active Compound.................. | 0.05-5 |
| Isopropyl Myristate.............. | 20 |
| Alcohol (Denatured) qs........... | 100 |

Example 72

Preparation of Buccal Tablet

| Ingredient | mg./Tablet |
|---|---|
| Active Ingredient..................... | 3.25 |
| 6 x Sugar............................. | 290.60 |
| Acacia................................ | 14.53 |
| Soluble Starch........................ | 14.53 |
| F. D. & C. Yellow No. 6 Dye............ | 0.49 |
| Magnesium Stearate.................... | 1.60 |
| | 325.00 |

The final tablet will weigh about 325 mg. and may be compressed into buccal tablets in flat faced or any other tooling shape convenient for buccal adminsitration.

## Example 73

### Preparation of Lozenge

| Ingredient | g./Lozenge |
|---|---|
| Active Ingredient | 0.0140 |
| Kompact® Sugar (Sucrest Co.) | 0.7138 |
| 6 x Sugar | 0.4802 |
| Sorbitol (USP Crystalline) | 0.1038 |
| Flavor | 0.0840 |
| Magnesium Stearate | 0.0021 |
| Dye | qs |
| Stearic Acid | 0.0021 |
| | 1.4000 |

The ingredients are compressed into 5/8" flat based lozenge tooling. Other shapes may also be utilized.

The compounds of the present invention may be administered internally, e.g., orally, intra-articularly or parenterally, to a warm-blooded animal to inhibit complement in the body fluid of the animal, such inhibition being useful in the amelioration or prevention of those reactions dependent upon the function of complement, such as inflammatory process and cell membrane damage induced by antigen-antibody complexes. A range of doses may be employed depending on the mode of administration, the condition being treated and the particular compound being used. For example, for intravenous or subcutaneous use from about 5 to about 50 mg/kg/day, or every six hours for more rapidly excreted salts, may be used. For intra-articular use for large joints such as the knee, from about 2 to about 20 mg/joint/week may be used, with proportionally smaller doses for smaller joints. The dosage range is to be adjusted to provide optimum therapeutic response in the warm-blooded animal being treated. In general, the amount of compound administered can vary over a wide range to provide from about 5 mg/kg to about 100 mg/kg of body weight of animal per day. The usual daily dosage for a 70 kg subject may vary from about 350 mg to about 3.5 g. Unit doses of the acid or salt can contain from about 0.5 mg to

about 500 mg.

The compounds of the present invention may also be administered topically in the form of ointments, creams, lotions and the like, suitable for the treatment of complement dependent dermatological disorders.

Moreover, the compounds of the present invention may be administered in the form of dental pastes, ointments, buccal tablets and other compositions suitable for application periodontally for the treatment of periodontitis and related diseases of the oral cavity.

In therapeutic use, the compounds of this invention may be administered in the form of conventional pharmaceutical compositions. Such compositions may be formulated so as to be suitable for oral or parenteral administration. The active ingredient may be combined in admixture with a pharmaceutically acceptable carrier, which carrier may take a wide variety of forms depending on the form of preparation desired for administration, i.e., oral or parenteral. The compounds can be used in compositions such as tablets. Here, the principal active ingredient is mixed with conventional tabletting ingredients such as corn starch, lactose, sucrose, sorbitol, talc, stearic acid, magnesium stearate, dicalcium phosphate, gums, or similar materials as nontoxic pharmaceutically acceptable diluents or carriers. The tablets or pills of the novel compositions can be laminated or otherwise compounded to provide a dosage form affording the advantage of prolonged or delayed action or predetermined successive action of the enclosed medication. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures

of polymeric acids with such materials as shellac, shellac and cetyl alcohol, cellulose acetate and the like. A particularly advantageous enteric coating comprises a styrene maleic acid copolymer together with known materials contributing to the enteric properties of the coating. The tablet or pill may be colored through the use of an appropriate nontoxic dye, so as to provide a pleasing appearance.

The liquid forms in which the novel compositions of the present invention may be incorporated for administration include suitable flavored emulsions with edible oils, such as, cottonseed oil, sesame oil, coconut oil, peanut oil, and the like, as well as elixirs and similar pharmaceutical vehicles. Sterile suspensions or solutions can be prepared for parenteral use. Isotonic preparations containing suitable preservatives are also desirable for injection use.

The term "dosage form," as described herein, refers to physically discrete units suitable as unitary dosage for warm-blooded animal subjects, each unit containing a predetermined quantity of active component calculated to produce the desired therapeutic effect in association with the required pharmaceutical diluent, carrier or vehicle. The specification for the novel dosage forms of this invention are indicated by characteristics of the active component and the particular therapeutic effect to be achieved or the limitations inherent in the art of compounding such an active component for therapeutic use in warm-blooded animals as disclosed in this specification. Examples of suitable oral dosage forms in accord with this invention are tablets, capsules, pills, powder packets, granules, wafers, cachets, teaspoonfuls, dropperfuls, ampules, vials, segregated multiples of any of the foregoing and other forms as herein described.

The complement inhibiting activity of the compounds of this invention has been demonstrated by one or more of the following identified tests: (i) Test Code 026 (Cl inhibitor) - This test measures the ability of activa-

ted human C1 to destroy fluid phase human C2 in the presence of C4 and appropriate dilutions of the test compound. An active inhibitor protects C2 from C1 and C4; (ii) Test Code 035 (C3-C9 inhibitor) - This test determines the ability of the late components of human complement (C3-C9) to lyse EAC 142 in the presence of appropriate dilutions of the test compound. An active inhibitor protects EAC 142 from lysis by human C3-C9; (iii) Cap 50 Test - Here, appropriate amounts of the test compound are added to a pool of guinea pig (or human) serum in vitro, after which the undiluted serum capillary tube assay of U.S. Patent No. 3,876,376 is run. The concentration of compound inhibiting 50% is reported; and (iv) Guinea Pig Intraperitoneal Test (GPIP) - Guinea pigs weighing about 300 g are dosed intraperitoneally (i.p.) with 200 mg/kg of the test compound dissolved in saline and adjusted to pH 7-8. Approximately 0.4 ml blood samples, taken by orbital sinus puncture 2 hours and 6 hours after injections, are collected directly into centrifuge tubes; 5 ml blood samples, taken by decapitation 24 hours after injection, are collected directly into beakers. The samples are allowed to clot, centrifuged, and the resultant sera are assayed for complement activity using the capillary complement assay. Percent inhibition is calculated by comparison with simultaneous controls. The results of the GPIP appear in Table IV together with results of Test Code 026, 035, and Cap 50. Table IV shows that the principal compounds of the invention possess highly significant complement modulating activity in warm-blooded animals.

TABLE IV

Biological Activities

| Compound | in vitro Activity | | | | in vivo Activity | | |
|---|---|---|---|---|---|---|---|
| | | | | | Guinea Pig % Inhibition Intraperitoneal Time (Hours) | | |
| | Cl 026* Wells | C-Late 035* Wells | Guinea Pig Cap 50 | Human Cap 50 | 2 | 6 | 24 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]octanediamide | 9** | 1.5** | 153 | 180 | 87 | 71 | 44 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]butanediamide | 10.2 | 1.4 | 236 | 40 | 85 | 83 | 34 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]pentanediamide | 9.9 | 1.5 | 156 | 58 | 90 | 88 | 44 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]hexanediamide | 10.5 | 1.0 | 56 | 94 | 90 | 85 | 76 |

TABLE IV (continued)

| Compound | in vitro Activity: Cl 026* Wells | in vitro Activity: C-Late 035* Wells | in vitro Activity: Guinea Pig Cap 50 | in vitro Activity: Human Cap 50 | in vivo Activity: Guinea Pig % Inhibition Intraperitoneal Time (Hours) 2 | 6 | 24 |
|---|---|---|---|---|---|---|---|
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]heptanediamide | 10.7 | 1.0 | 183 | 95 | 87 | 87 | 50 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]decanediamide | 5.0 | 0.8 | 121 | 394 | 88 | 87 | 75 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]propanediamide | 9.9 | 1.6 | 158 | 84 | 90 | 90 | 47 |
| Tetradecasodium N,N'-bis[4-[2,3,6-tri-O-sulfo-4-O-(2,3,4,6-tetra-O-sulfo-β-D-glucopyranosyl)-β-D-glucopyranosylthio]-phenyl]pentanediamide | 12 | 2.0 | | | | | |

TABLE IV (continued)

| Compound | in vitro Activity | | | | in vivo Activity | | |
|---|---|---|---|---|---|---|---|
| | Cl 026* Wells | C-Late 035* Wells | Guinea Pig Cap 50 | Human Cap 50 | Guinea Pig % Inhibition Intraperitoneal Time (Hours) 2 | 6 | 24 |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]tetradecanediamide | 7 | 3.0 | | | | | |
| Tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]ethanediamide | 9.5 | 1.2 | 213 | 124 | 90 | 90 | 80 |

TABLE IV (continued)

| Compound | Cl 026* Wells | C-Late 035* Wells |
|---|---|---|
| Tetradecasodium N,N'-bis[4-[2,3,6-tri-O-sulfo-4-O-(2,3,4,6-tetra-O-sulfo-α-D-glucopyranosyl)-β-D-glucopyranosyloxy]-phenyl]butanediamide | 11** | 1.0** |
| Tetradecasodium N,N'-bis[4-[2,3,6-tri-O-sulfo-4-O-(2,3,4,6-tetra-O-sulfo-α-D-glucopyranosyl)-β-D-glucopyranosyloxy]-phenyl]butanediamine | 12 | 2.0 |
| Octasodium N,N'-bis[4-[(2,3,4,6-tetra-O-sulfo-β-D-glucopyranosyl)thio]phenyl]pentanediamide | 7 | |

TABLE IV (continued)

| Compound | In vitro Activity: C-1 026* Wells | In vitro Activity: C-Late 035* Wells | In vitro Activity: Guinea Pig Cap* 50 | In vitro Activity: Human Cap* 50 | In vivo Activity (Guinea Pig % Inhibition) (Intraperitoneal) Time (Hours): 2 | 6 | 24 |
|---|---|---|---|---|---|---|---|
| Tetradecasodium N,N'-bis[4-[[2,3-6-tri-O-sulfo-4-O-(2,3,4,6-tetra-O-sulfo-α-D-glucopyranosyl)-β-D-glucopyranosyl]thio]-phenyl]urea | 11** | 2 | 133 | 85 | 90 | 91 | 58 |

* Tests identified by code herein.

**Activity in wells, a serial dilution assay; higher well number indicates higher activity. The serial dilutions are two-fold.

WE CLAIM:

1. A compound of the formula:

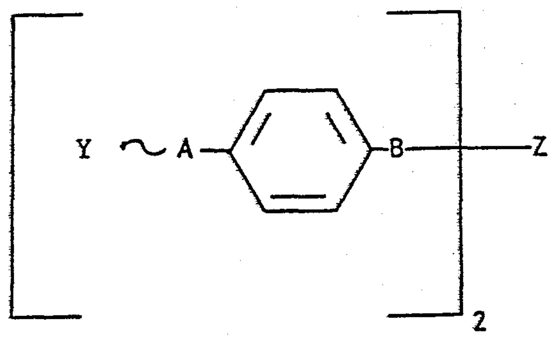

(∼ = α or β)

wherein Y is 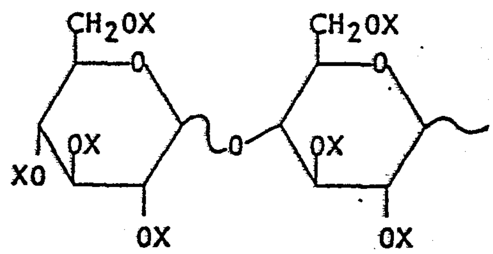

or 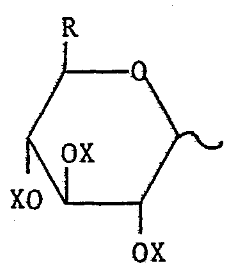 ;

X is $-SO_3M$ and M is a nontoxic pharmaceutically acceptable cation salt, wherein the salt forming moiety is alkali metal, alkaline earth metal, aluminum, ammonia or substituted ammonia consisting of trialkylamine ($C_1$-$C_6$), piperidine, pyrazine, alkanolamine ($C_2$-$C_6$) or cycloalkylamine ($C_3$-$C_6$); A is -S- or -O-; B is -NHCO-, $-NHSO_2-$, $-NHCH_2-$ or -NH-; R is $CH_2OSO_3M$, $COOCH_3$ or COOM; and Z is -CO- or a straight or branched chain alkylidene group $-(CH_2)_m-$, where m is an integer 0-12, inclusive, with the proviso that when m is zero or one, B cannot be $-NHSO_2-$.

2. The compound according to Claim 1, tetradecasodium N,N'-bis[4-[2,3,6-tri-O-sulfo-4-O-(2,3,4,6-tetra-O--sulfo-α-D-glucopyranosyl)-β-D-glucopyranosylthio]phenyl]-octanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]butanediamide; tetradecasodium N,N'-bis[4-[2,3,6-tri-O-sulfo-4-O-(2,3,4,6-tetra-O-sulfo-α--D-glucopyranosyl)-β-D-glucopyranosylthio]phenyl]pentanediamide; tetradecasodium N,N'-bis[4-[2,3,6-tri-O-sulfo-4--O-(2,3,4,6-tetra-O-sulfo-α-D-glucopyranosyl)-β-D-glucopyranosylthio]phenyl]hexanediamide; tetradecasodium N,N'-bis-[4-[2,3,6-tri-O-sulfo-4-O-(2,3,4,6-tetra-O-sulfo-α-D-glucopyranosyl)-β-D-glucopyranosylthio]phenyl]heptanediamide; tetradecasodium N,N'-bis[4-[2,3,6-tri-O-sulfo-4-O-(2,3,4,6--tetra-O-sulfo-α-D-glucopyranosyl)-β-D-glucopyranosylthio]-phenyl]decanediamide; tetradecasodium N,N'-bis[4-[2,3,6--tri-O-sulfo-4-O-(2,3,4,6-tetra-O-sulfo-α-D-glucopyranosyl)--β-D-glucopyranosylthio]phenyl]propanediamide; tetradecasodium N,N'-bis[4-[2,3,6-tri-O-sulfo-4-O-(2,3,4,6-tetra-O--sulfo-β-D-glucopyranosyl)-β-D-glucopyranosylthio]phenyl]-pentanediamide; tetradecasodium N,N'-bis[4-[2,3,6-tri-O--sulfo-4-O-(2,3,4,6-tetra-O-sulfo-α-D-glucopyranosyl)-β-D--glucopyranosylthio]phenyl]tetradecanediamide; tetradecasodium N,N'-bis[4-[2,3,6-tri-O-sulfo-4-O-(2,3,4,6-tetra-O--sulfo-α-D-glucopyranosyl)-β-D-glucopyranosylthio]phenyl]-ethanediamide; tetradecasodium N,N'-bis[4-[2,3,6-tri-O--sulfo-4-O-(2,3,4,6-tetra-O-sulfo-α-D-glucopyranosyl)-β-D--glucopyranosylthio]phenyl]butanediamine; tetradecatriethylammonium N,N'-bis[4-[2,3,6-tri-O-sulfo-4-O-(2,3,4,6-tetra--O-sulfo-α-D-glucopyranosyl)-β-D-glucopyranosylthio]phenyl]-octanediamide; tetradecatriethylammonium N,N'-bis[4-[2,3,6--tri-O-sulfo-4-O-(2,3,4,6-tetra-O-sulfo-α-D-glucopyranosyl)--β-D-glucopyranosylthio]phenyl]butanediamide; tetradecatriethylammonium N,N'-bis[4-[2,3,6-tri-O-sulfo-4-O-(2,3,4,6--tetra-O-sulfo-α-D-glucopyranosyl)-β-D-glucopyranosylthio]-phenyl]pentanediamide; tetradecatriethylammonium N,N'-bis-[4-[2,3,6-tri-O-sulfo-4-O-(2,3,4,6-tetra-O-sulfo-α-D-glucopyranosyl)-β-D-glucopyranosylthio]phenyl]hexanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]heptanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosyl]thio]phenyl]-decanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]propanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]-phenyl]tetradecanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]ethanediamide; tetradecatriethylammonium N,N'-bis[4-[2,3,6-tri-O-sulfo-4-O-(2,3,4,6-tetra-O-sulfo-β-D-glucopyranosyl)-β-D-glucopyranosylthio]phenyl]pentanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]-phenyl]butanediamine; tetradecasodium N,N'-bis[4-[2,3,6-tri-O-sulfo-4-O-(2,3,4,6-tetra-O-sulfo-α-D-glucopyranosyl)-β-D-glucopyranosyloxy]phenyl]butanediamide; tetradecasodium N,N'-bis[4-[2,3,6-tri-O-sulfo-4-O-(2,3,4,6-tetra-O-sulfo-α-D-glucopyranosyl)-β-D-glucopyranosyloxy]phenyl]-butanediamine; tetradecatriethylammonium N,N'-bis[4-[2,3,6-tri-O-sulfo-4-O-(2,3,4,6-tetra-O-sulfo-α-D-glucopyranosyl)-β-D-glucopyranosyloxy]phenyl]butanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosyloxy]-phenyl]butanediamine; octasodium N,N'-bis[4-[(2,3,4,6-tetra-O-sulfo-β-D-glucopyranosyl)thio]phenyl]pentanediamide; hexasodium (1,4-dioxo-1,4-butanediyl)bis(imino-4,1-phenylene)bis(2,3,4-tri-O-sulfo-1-thio-β-D-glucopyranosiduronic acid), dimethyl ester; octasodium (1,5-dioxo-1,5-pentanediyl)bis(imino-4,1-phenylene)bis(2,3,4-tri-O-sulfo-1-thio-β-D-glucopyranosiduronic acid); octatriethylammonium N,N'-bis[4-[(2,3,4,6-tetra-O-sulfo-β-D-glucopyranosyl)thio]-phenyl]pentanediamide; hexatriethylammonium (1,4-dioxo-1,4--butanediyl)bis(imino-4,1-phenylene)bis(2,3,4-tri-O-sulfo--1-thio-β-D-glucopyranosiduronic acid), dimethyl ester; octatriethylammonium (1,5-dioxo-1,5-pentanediyl)bis(imino--4,1-phenylene)bis(2,3,4-tri-O-sulfo-1-thio-β-D-glucopyranosiduronic acid); tetradecatriethylammonium N,N'-bis[4--[[2,3,6-tri-O-sulfo-4-O-(2,3,4,6-tetra-O-sulfo-α-D-glucopyranosyl)-β-D-glucopyranosyl]thio]phenyl]urea; or tetradecasodium N,N'-bis[4-[[2,3,6-tri-O-sulfo-4-O-(2,3,4,6--tetra-O-sulfo-α-D-glucopyranosyl)-β-D-glucopyranosyl]thio]-phenyl]urea.

3. A process of preparing a compound of the formula:

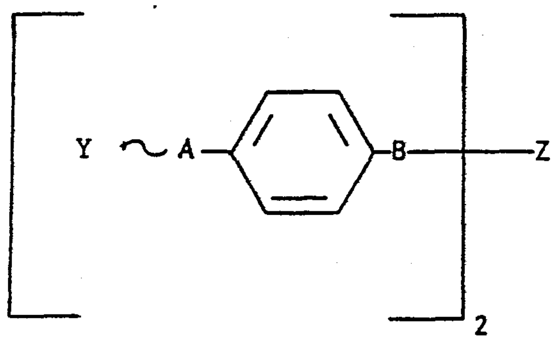

(~ = α or β)

wherein Y is

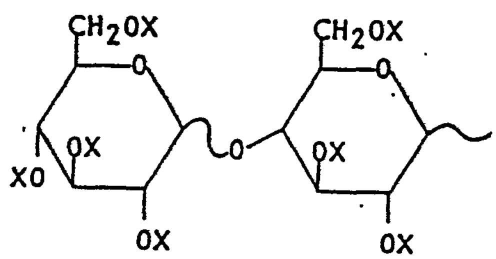

or

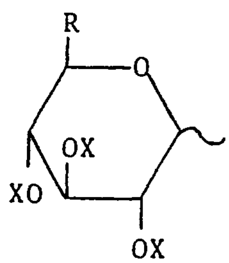

;

X is $-SO_3M$ and M is a nontoxic pharmaceutically acceptable cation salt, wherein the salt forming moiety is alkali metal, alkaline earth metal, aluminum, ammonia or substituted ammonia consisting of trialkylamine ($C_1$-$C_6$), piperidine, pyrazine, alkanolamine ($C_2$-$C_6$) or cycloalkylamine ($C_3$-$C_6$); A is -S- or -O-; B is -NHCO-, $-NHSO_2-$, $-NHCH_2-$ or -NH-; R is $CH_2OSO_3M$, $COOCH_3$ or COOM; and Z is -CO- or a straight or branched chain alkylidene group $-(CH_2)_m-$, where m is an integer 0-12, inclusive, with the proviso that when m is zero or one, B cannot be $-NHSO_2-$, which comprises: (A) reacting 4-aminothiophenyl in dimethoxyethane and sodium hydride in oil with a solution of acetobromo-α-D-maltose or cellobiose in dimethoxyethane, under an inert atmosphere, for several hours, subjecting the mother liquor to dry column chromatography on silica gel using the solvent system ethyl acetate:hexane (1:1) giving 4-aminophenyl 2,3,6-tri-O--acetyl-4-O-(2,3,4,6-tetra-O-acetyl-α(or β)-D-glucopyranosyl-1-thio-α(or β)-D-glucopyranoside; then reacting with an appropriate acid chloride of the formula $X-(CH_2)_m-X$, where X is COCl or $SO_2Cl$ and m is an integer 0-12 (with the proviso that when m is zero or one X cannot be $SO_2Cl$), in dry pyridine:acetonitrile (1:1) under an inert atmosphere for 18-36 hours, extracting from a suitable organic solvent, giving a compound of the formula:

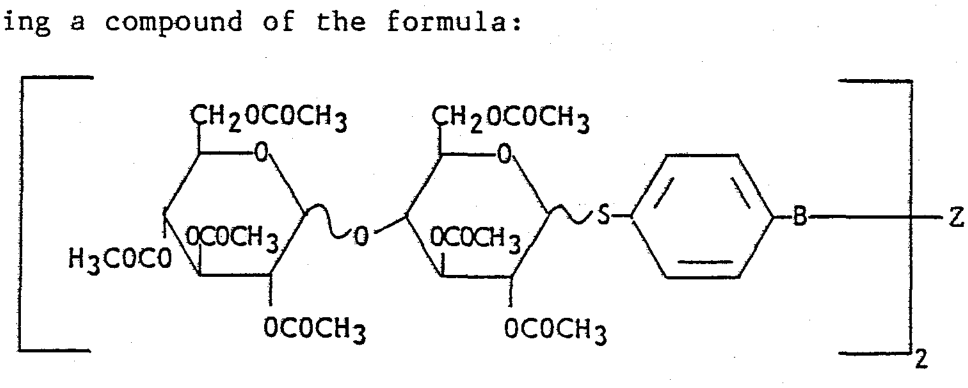

(~ = α or β)

where B and Z are as hereinabove defined, treating this acetylated derivative in ammonia saturated methanol at 0°C, or sodium in methanol, for 18-24 hours; extracting from

absolute ethanol, giving the corresponding deacetylated derivative of the formula:

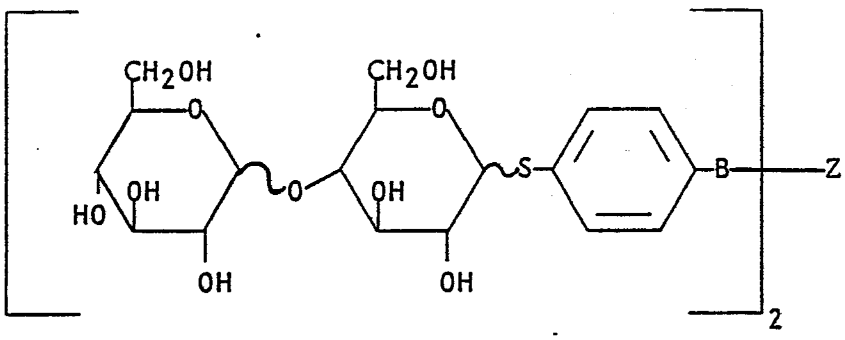

(~ = α or β)

where B and Z are as hereinabove defined, then converting to the tetradecatriethylammonium sulfate derivatives of the formula:

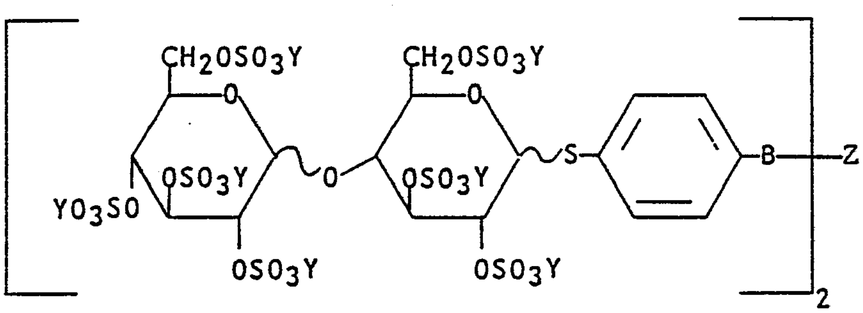

(~ = α or β)

where B and Z are as hereinabove defined and Y is $\overset{+}{N}H(C_2H_5)_3$, by treating with triethylamine-sulfur trioxide complex in N,N-dimethylacetamide, under an inert atmosphere at 60-65°C, for several hours; reacting with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitating in ethanol, giving the desired products; or (B)

reacting sodium 4-nitrophenol and potassium hydroxide with a solution of acetobromo-α-D-maltose or cellobiose in aqueous acetone for several hours, then reducing the resultant product in a hydrogen atmosphere in the presence of palladium-on-carbon and acetic acid as solvent, giving 4-aminophenyl 2,3,6-tri-O-acetyl-4-O-(2,3,4,6-tetra-O-acetyl-α(or β)-D-glucopyranosyl)-α(or β)-D-glucopyranoside; reacting with an appropriate acid chloride of the formula $X-(CH_2)_m-X$, where X is COCl or $SO_2Cl$ and m is an integer 0-12 (with the proviso that when m is zero or one, X cannot be $SO_2Cl$), in dry pyridine:acetonitrile (1:1) under an inert atmosphere for 18-36 hours; extracting from a suitable organic solvent, giving a compound of the formula:

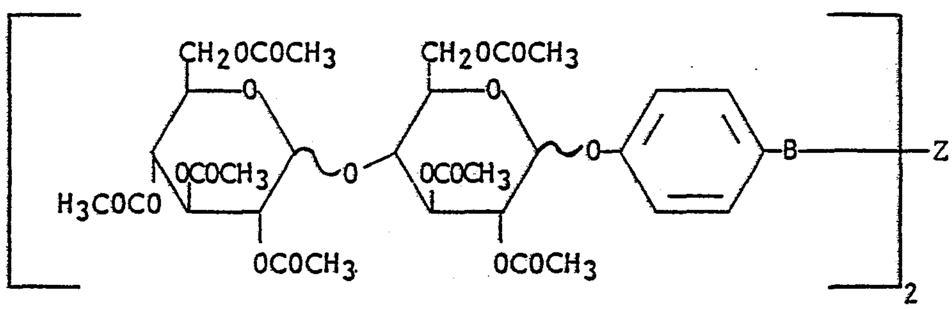

(~ = α or β)

where B and Z are as hereinabove defined; treating this acetylated derivative in ammonia-saturated methanol at 0°C, or sodium in methanol, for 18-24 hours; giving the corresponding deacetylated derivative of the formula:

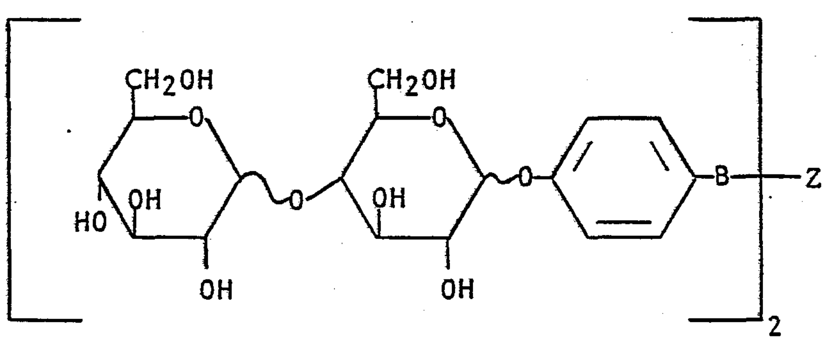

(~ = α or β)

where B and Z are as hereinabove defined; then converting to the tetradecatriethylammonium sulfate derivative of the formula:

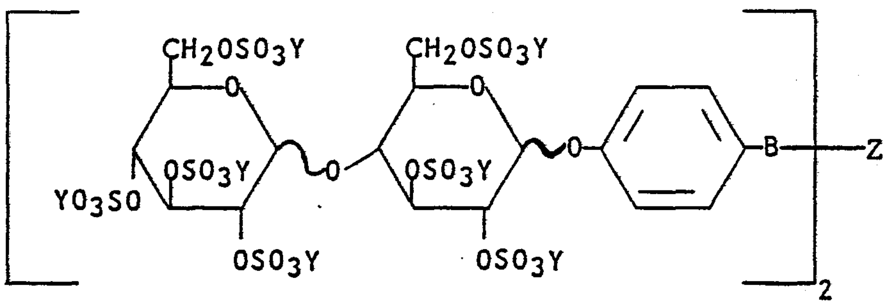

(~ = α or β )

where Y is $\overset{+}{N}H(C_2H_5)_3$, by treating with triethylamine-sulfur trioxide complex in N,N-dimethylacetamide, under an inert atmosphere at 60-65°C, for several hours; reacting with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitating in ethanol, giving the desired products ; or (C) either reacting a mixture of sodium hydride in oil dispersion in a solvent such as dimethoxyethane under an inert atmosphere with a solution of aminothiophenol in dimethoxyethane; stirring for 3.5 hours; adding a solution of a bromoacetyl pyranoside in dimethoxyethane; stirring overnight; giving 4-aminophenylthio 2,3,4,6-tetra-O--acetyl-α(or β)-D-glucopyranoside derivative; or alternatively, reacting a mixture of sodium hydride in oil dispersion in a solvent such as dimethoxyethane under an inert atmosphere with a solution of sodium aminothiophenol in dimethoxyethane; stirring for 2 hours; adding a solution of methyl acetobromoglucuronate in dimethoxyethane, stirring overnight; giving 4-aminophenylthio 2,3,4-tri-O-acetyl-α(or β)-D-glucopyranosiduronic acid, methyl ester derivative; then reacting either 4-aminophenylthio 2,3,4,6-tetra-O-acetyl-α(or β)-D-glucopyranoside or 4-aminophenylthio 2,3,4-

-tri-O-acetyl-α(or β)-D-glucopyranosiduronic acid, methyl ester with an acid chloride, for example, 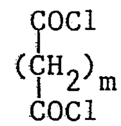

where m is an integer 0-12 in a solvent such as acetonitrile under an inert atmosphere for 2 or 4 hours respectively; reacting this acetate with ammonia-saturated methanol at -5° to +5°C or with sodium in methanol under an inert atmosphere for 2 hours; then reacting with triethylamine-sulfur trioxide in N,N-dimethylacetamide at 60-65°C under an inert atmosphere for 4 hours, giving the polytriethylammonium derivative of the above formula where M is $NH^+(C_2H_5)_3$; then reacting with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and precipitating in ethanol, giving the final products; or (D) reacting a 4-aminophenyl 2,3,6-tri-O-acetyl-4-O-(2,3,4,6-tetra-O-acetyl-α(or β)-D-glucopyranosyl)-1-thio-α(or β)-D-glucopyranoside with phosgene in toluene and pyridine for 15-25 hours and purifying by chromatography, giving an N,N'-bis[4-[[2,3,6-tri-O-acetyl-4-O-(2,3,4,6-tetra-O-acetyl-α(or β)-D-glucopyranosyl)-α(or β)-D-glucopyranosyl]thio]phenyl]urea; then reacting with methanol saturated with ammonia at -5 to +5°C, giving an N,N'-bis[4-[[4-O-(α(or β)-D-glucopyranosyl)-α(or β)-D-glucopyranosyl]thio]phenyl]urea; then reacting with triethylamine-sulfur trioxide in dry N,N-dimethylacetamide at 60-70°C for 40-60 hours giving the compounds of the above formula where M is $\overset{+}{N}H(C_2H_5)_3$; reacting with a cation-containing compound wherein the salt forming moiety is selected from the group consisting of alkali metal, alkaline earth metal, aluminum, ammonia and substituted ammonia selected from the group consisting of piperidine, pyrazine, alkanolamine ($C_2$-$C_6$) and cycloalkylamine ($C_3$-$C_6$), and thereafter precipitating in ethanol, giving the desired products.

4. A composition of matter in dosage unit form which comprises an effective complement modulating amount from about 0.5 mg to about 500 mg of a compound of the formula:

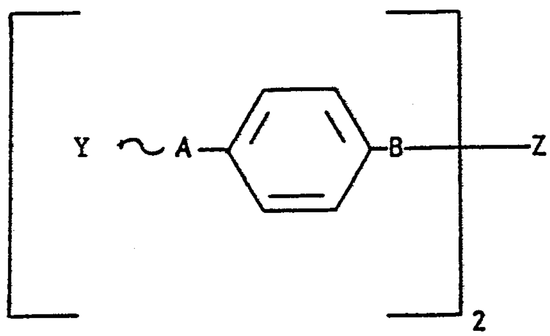

(~ = α or β)

wherein Y is

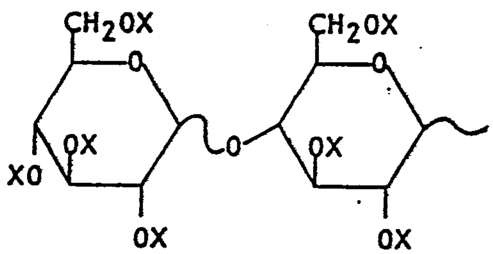

or

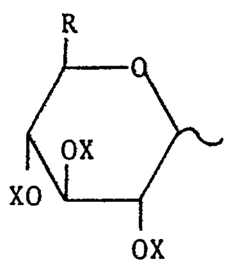

;

X is $-SO_3M$ and M is a nontoxic pharmaceutically acceptable cation salt, wherein the salt forming moiety is alkali metal, alkaline earth metal, aluminum, ammonia or substituted ammonia consisting of trialkylamine ($C_1$-$C_6$), piperidine, pyrazine, alkanolamine ($C_2$-$C_6$) or cycloalkylamine ($C_3$-$C_6$); A is -S- or -O-; B is -NHCO-, $-NHSO_2-$, $-NHCH_2-$ or -NH-; R is $CH_2OSO_3M$, $COOCH_3$ or COOM; and Z is -CO- or a straight or branched chain alkylidene group $-(CH_2)_m-$, where m is an integer 0-12, inclusive, with the proviso that when m is zero or one, B cannot be $-NHSO_2-$; in association with a pharmaceutically acceptable carrier.

5. The composition of matter according to Claim 4, wherein the compound is tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]-octanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]butanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]pentanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]hexanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]heptanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]-phenyl]decanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]propanediamide; tetradecasodium N,N'-bis[4-[2,3,6-tri-O-sulfo-4-O-(2,3,4,6-tetra-O-sulfo-β-D-glucopyranosyl)-β-D-glucopyranosylthio]phenyl]-pentanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]tetradecanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]-ethanediamide; tetradecasodium N,N'-bis[4-[2,3,6-tri-O-sulfo-4-O-(2,3,4,6-tetra-O-sulfo-α-D-glucopyranosyl)-β-D-glucopyranosylthio]phenyl]butanediamine; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]-octanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]butanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]-

phenyl]pentanediamide; tetradecatriethylammonium N,N'-bis-[4-[2,3,6-tri-O-sulfo-4-O-(2,3,4,6-tetra-O-sulfo-α-D-glucopyranosyl)-β-D-glucopyranosylthio]phenyl]hexanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]heptanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosyl]thio]phenyl]-decanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]propanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]-phenyl]tetradecanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]phenyl]ethanediamide; tetradecatriethylammonium N,N'-bis[4-[2,3,6-tri-O-sulfo-4-O-(2,3,4,6-tetra-O-sulfo-β-D-glucopyranosyl)-β-D-glucopyranosylthio]phenyl]pentanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosylthio]-phenyl]butanediamine; tetradecasodium N,N'-bis[4-[2,3,6-tri-O-sulfo-4-O-(2,3,4,6-tetra-O-sulfo-α-D-glucopyranosyl)-β-D-glucopyranosyloxy]phenyl]butanediamide; tetradecasodium N,N'-bis[4-[2,3,6-tri-O-sulfo-4-O-(2,3,4,6-tetra-O-sulfo-α-D-glucopyranosyl)-β-D-glucopyranosyloxy]phenyl]-butanediamine; tetradecatriethylammonium N,N'-bis[4-[2,3,6-tri-O-sulfo-4-O-(2,3,4,6-tetra-O-sulfo-α-D-glucopyranosyl)-β-D-glucopyranosyloxy]phenyl]butanediamide; tetradecatriethylammonium N,N'-bis[4-[2,3,6-tri-O-sulfo-4-O-(2,3,4,6-tetra-O-sulfo-α-D-glucopyranosyl)-β-D-glucopyranosyloxy]-phenyl]butanediamine; octasodium N,N'-bis[4-[(2,3,4,6-tetra-O-sulfo-β-D-glucopyranosyl)thio]phenyl]pentanediamide; hexasodium (1,4-dioxo-1,4-butanediyl)bis(imino-4,1-phenylene)bis(2,3,4-tri-O-sulfo-1-thio-β-D-glucopyranosiduronic acid), dimethyl ester; octasodium (1,5-dioxo-1,5-pentanediyl)bis(imino-4,1-phenylene)bis(2,3,4-tri-O-sulfo-1-thio-

-β-D-glucopyranosiduronic acid); octatriethylammonium N,N'-bis[4-[(2,3,4,6-tetra-O-sulfo-β-D-glucopyranosyl)thio]phenyl]pentanediamide; hexatriethylammonium (1,4-dioxo-1,4-butanediyl)bis(imino-4,1-phenylene)bis(2,3,4-tri-O-sulfo-1-thio-β-D-glucopyranosiduronic acid), dimethyl ester; octatriethylammonium (1,5-dioxo-1,5-pentanediyl)bis(imino-4,1-phenylene)bis(2,3,4-tri-O-sulfo-1-thio-β-D-glucopyranosiduronic acid); tetradecatriethylammonium N,N'-bis[4-[[2,3,6-tri-O-sulfo-4-O-(2,3,4,6-tetra-O-sulfo-α-D-glucopyranosyl)-β-D-glucopyranosyl]thio]phenyl]urea; or tetradecasodium N,N'-bis[4-[[2,3,6-tri-O-sulfo-4-O-(2,3,4,6-tetra-O-sulfo-α-D-glucopyranosyl)-β-D-glucopyranosyl]thio]phenyl]urea.

6. A compound of the formula:

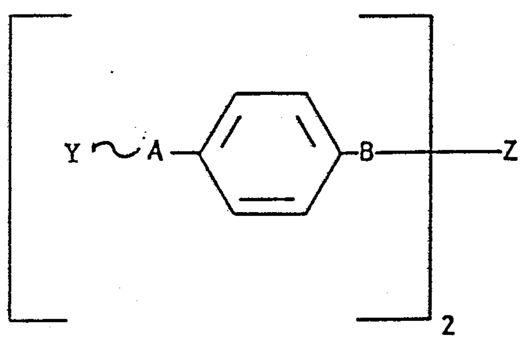

(~ = α or β)

wherein Y is

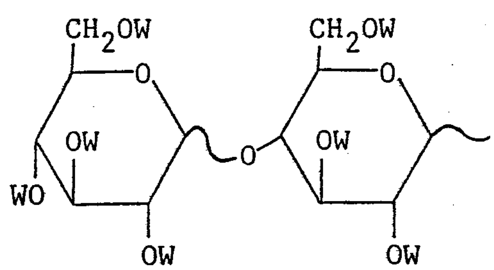

or

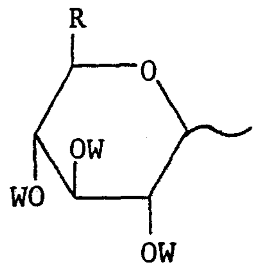

;

W is -H or $-COCH_3$; R is $-CH_2OW$; -COOH or $-COOCH_3$; A is -S- or -O-; B is -NHCO-, $-NHSO_2-$, $-NHCH_2-$ or -NH-; and Z is -CO- or a straight or branched chain alkylidene group $-(CH_2)_m-$, where m is an integer 0-12, inclusive, with the proviso that when m is zero or one, B cannot be $-NHSO_2-$.

7. A dental preparation characterized in that said preparation contains as a topically effective, therapeutically active ingredient a compound of the formula:

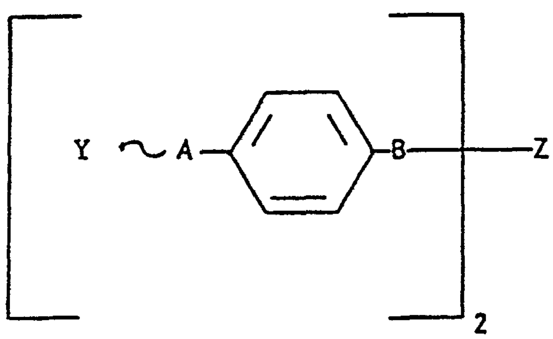

(∼ = α or β)

wherein Y is

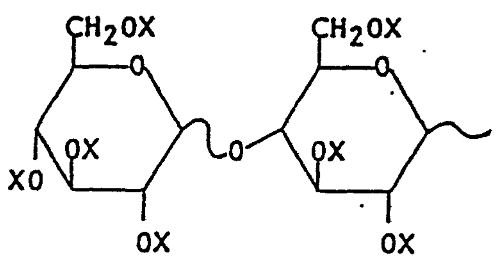

or

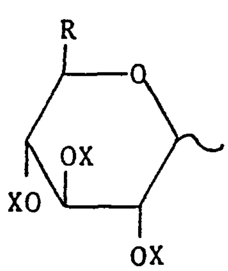

;

X is $-SO_3M$ and M is a nontoxic pharmaceutically acceptable cation salt, wherein the salt forming moiety is alkali metal, alkaline earth metal, aluminum, ammonia or substituted ammonia consisting of trialkylamine ($C_1$-$C_6$), piperidine, pyrazine, alkanolamine ($C_2$-$C_6$) or cycloalkylamine ($C_3$-$C_6$); A is -S- or -O-; B is -NHCO-, $-NHSO_2-$, $-NHCH_2-$ or -NH-; R is $CH_2OSO_3M$, $COOCH_3$ or COOM; and Z is -CO- or a straight or branched chain alkylidene group $-(CH_2)_m-$, where m is an integer 0-12, inclusive, with the proviso that when m is zero or one, B cannot be $-NHSO_2-$.

0092037

European Patent Office

# EUROPEAN SEARCH REPORT

Application number

EP 83 10 1634

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-4 221 907 (V.G. NAIR)<br>* Claims 1-4 *<br>--- | 1,7 | C 07 H 15/20<br>C 07 H 3/04<br>A 61 K 31/70<br>A 61 K 7/16 |
| A | US-A-4 247 535 (A.J. LEWIS)<br>* Claims 1-4 *<br>--- | 1,7 | |
| P,A | US-A-4 337 249 (R.B. CONROW)<br>* Claims 1-4 *<br>----- | 1,7 | |
| | | | TECHNICAL FIELDS SEARCHED (Int. Cl. 3) |
| | | | C 07 H 15/00<br>C 07 H 3/00<br>A 61 K 31/00 |

The present search report has been drawn up for all claims

| Place of search | Date of completion of the search | Examiner |
|---|---|---|
| THE HAGUE | 16-05-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82